Europäisches Patentamt
European Patent Office
Office européen des brevets

(11) Veröffentlichungsnummer: **0 300 371**
**A1**


(12) # EUROPÄISCHE PATENTANMELDUNG


(21) Anmeldenummer: **88111347.6**

(22) Anmeldetag: **14.07.88**

(51) Int. Cl.⁴: **C07C 103/46 , C07C 103/30 , C07C 103/84 , C07C 103/49 , C07D 225/06 , C07D 213/30 , C07D 213/75 , C07D 295/12 , C07C 127/17 , A61K 31/165 , A61K 31/44**

Patentansprüche für folgende Vertragsstaaten: ES + GR.

(30) Priorität: **21.07.87 CH 2764/87**

(43) Veröffentlichungstag der Anmeldung: **25.01.89 Patentblatt 89/04**

(84) Benannte Vertragsstaaten: **AT BE CH DE ES FR GR IT LI LU NL SE**



(71) Anmelder: **F. HOFFMANN-LA ROCHE & CO. Aktiengesellschaft**

**CH-4002 Basel(CH)**

(72) Erfinder: **Aschwanden, Werner**
**Greilingerstrasse 4**
**Ch-4107 Ettingen(CH)**
Erfinder: **Imhof, René, Dr.**
**Bleumatthöhe 3**
**Ch-5264 Gipf-Oberfrick(CH)**
Erfinder: **Jacob-Roetne, Roland, Dr.**
**Oberer Baselblick 26**
**D-7854 Inzlingen(DE)**
Erfinder: **Kyburz, Emilio, Dr.**
**Unterer Rebberweg 127**
**CH-4153 Reinach(CH)**

(74) Vertreter: **Lederer, Franz, Dr. et al**
**Van der Werth, Lederer & Riederer**
**Patentanwälte Lucile-Grahn-Strasse 22**
**D-8000 München 80(DE)**


(54) **Hydrozimtsäurederivate.**


(57) Hydrozimtsäurederivate der allgemeinen Formel

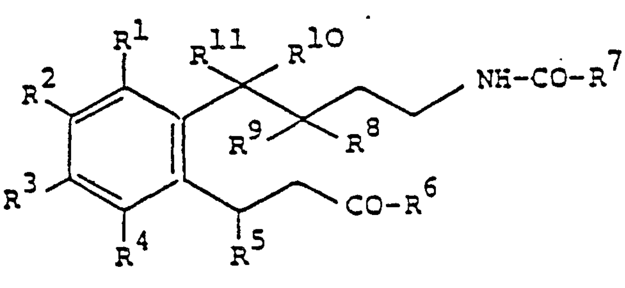

I

worin



EP 0 300 371 A1

- von den Symbolen $R^1$ bis $R^4$ eines oder zwei Halogen oder Methoxy und die übrigen Wasserstoff;
- $R^5$ Wasserstoff oder Phenyl;
- $R^6$ einen Rest der Formel $-OR^{12}$ (a) oder $-NR^{13}R^{14}$ (b);
- $R^7$ ($C_1$-$C_4$)-Alkyl, ($C_2$-$C_5$)-Alkanoylamino-($C_2$-$C_5$)-alkyl, Amino oder ($C_1$-$C_4$)-Alkoxyphenyl;
- $R^8$ und $R^9$ je Wasserstoff oder ($C_1$-$C_4$)-Alkyl;
- $R^{10}$ Wasserstoff und $R^{11}$ Hydroxy oder $R^{10}$ und $R^{11}$ zusammen Oxo;
- $R^{12}$ Wasserstoff, ($C_1$-$C_{10}$)-Alkyl, Pyridylmethyl oder Carbamoylmethyl;
- $R^{13}$ Wasserstoff, ($C_1$-$C_4$)-Alkyl und $R^{14}$ Wasserstoff, ($C_1$-$C_4$)-Alkyl, Pyridyl, Phenyl-($C_1$-$C_4$)-alkyl, Carboxy-($C_1$-$C_4$)-alkyl, Carbamoyl-($C_1$-$C_4$)-alkyl, ($C_1$-$C_4$)-Alkoxycarbonyl-($C_1$-$C_4$)-alkyl, Di-($C_1$-$C_4$)-alkoxycarbonyl-($C_2$-$C_5$)-alkyl, Piperidino-($C_2$-$C_4$)-alkyl oder Halopyridincarboxamido-($C_2$-$C_4$)-alkyl oder $R^{13}$ und $R^{14}$ zusammen mit dem Stickstoffatom 4-($C_1$-$C_4$)-Alkyl-piperazin-1-yl bedeuten,

sowie pharmazeutisch anwendbare Salze von basischen Verbindungen der Formel I mit Säuren bzw. von sauren Verbindungen der Formel I mit Basen eignen sich zur Bekämpfung bzw. Verhütung der cerebralen Insuffizienz bzw. zur Verbesserung cognitiver Funktionen.

## Hydrozimtsäurederivate

Die vorliegende Erfindung betrifft Hydrozimtsäurederivate. Im speziellen betrifft sie Hydrozimtsäurederivate der allgemeinen Formel

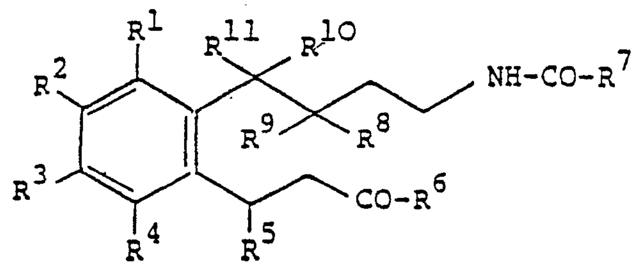

I

worin
- von den Symbolen $R^1$ bis $R^4$ eines oder zwei Halogen oder Methoxy und die übrigen Wasserstoff;
- $R^5$ Wasserstoff oder Phenyl;
- $R^6$ einen Rest der Formel $-OR^{12}$ (a) oder $-NR^{13}R^{14}$ (b);
- $R^7$ $(C_1-C_4)$-Alkyl, $(C_2-C_5)$-Alkanoylamino-$(C_2-C_5)$-alkyl, Amino oder $(C_1-C_4)$-Alkoxyphenyl;
- $R^8$ und $R^9$ je Wasserstoff oder $(C_1-C_4)$-Alkyl;
- $R^{10}$ Wasserstoff und $R^{11}$ Hydroxy oder $R^{10}$ und $R^{11}$ zusammen Oxo;
- $R^{12}$ Wasserstoff, $(C_1-C_{10})$-Alkyl, Pyridylmethyl oder Carbamoylmethyl;
- $R^{13}$ Wasserstoff, $(C_1-C_4)$-Alkyl und $R^{14}$ Wasserstoff, $(C_1-C_4)$-Alkyl, Pyridyl, Phenyl-$(C_1-C_4)$-alkyl, Carboxy-$(C_1-C_4)$-alkyl, Carbamoyl-$(C_1-C_4)$-alkyl, $(C_1-C_4)$-Alkoxycarbonyl-$(C_1-C_4)$-alkyl, Di-$(C_1-C_4)$-alkoxycarbonyl-$(C_2-C_5)$-alkyl, Piperidino-$(C_2-C_4)$-alkyl oder Halopyridincarboxamido-$(C_2-C_4)$-alkyl oder $R^{13}$ und $R^{14}$ zusammen mit dem Stickstoffatom 4-$(C_1-C_4)$-Alkyl-piperazin-1-yl bedeuten,

sowie pharmazeutisch anwendbare Salze von basischen Verbindungen der Formel I mit Säuren bzw. von sauren Verbindungen der Formel I mit Basen.

Diese Verbindungen und Salze sind neu und zeichnen sich durch wertvolle pharmakodynamische Eigenschaften aus.

Gegenstand der vorliegenden Erfindung sind Verbindungen der allgemeinen Formel I und pharmazeutisch anwendbare Salze davon als solche und als pharmazeutische Wirkstoffe, Verfahren zur Herstellung dieser Verbindungen und Salze, ferner diese enthaltende Arzneimittel und die Herstellung solcher Arzneimittel, sowie die Verwendung von Verbindungen der allgemeinen Formel I und von pharmazeutisch anwendbaren Salzen davon bei der Bekämpfung bzw. Verhütung von Krankheiten bzw. bei der Verbesserung der Gesundheit, insbesondere bei der Bekämpfung bzw. Verhütung der cerebralen Insuffizienz bzw. bei der Verbesserung cognitiver Funktionen und die Verwendung von Verbindungen der allgemeinen Formel I und von pharmazeutisch anwendbaren Salzen davon zur Herstellung von Arzneimitteln für die Bekämpfung bzw. Verhütung der cerebralen Insuffizienz bzw. für die Verbesserung cognitiver Funktionen.

Der in der vorliegenden Beschreibung verwendete Ausdruck "Alkyl" bezeichnet geradkettige oder verzweigte gesättigte Kohlenwasserstoffreste, wie Methyl, Aethyl, n-Propyl, Isopropyl, n-Butyl, Isobutyl, s-Butyl, t-Butyl und dergleichen. Der Ausdruck "Alkoxy" bezeichnet einen über ein Sauerstoffatom gebundenen Alkylrest im Sinne der vorstehenden Definition. Der Ausdruck "Alkanoyl" bezeichnet Reste, welche sich von Alkancarbonsäuren durch Elimination der Hydroxylgruppe ableiten und umfasst demnach Reste wie Acetyl und dergleichen. Der Ausdruck "Di-Alkoxycarbonylalkyl" bezeichnet Alkylreste, welche durch zwei, jedoch nicht am gleichen Kohlenstoffatom sitzende Alkoxycarbonylgruppen substituiert sind.

$R^4$ kann zweckmässigerweise Wasserstoff bedeuten, wobei zweckmässigerweise entweder $R^1$ Chlor und $R^2$ und $R^3$ Wasserstoff oder $R^1$ Wasserstoff und $R^2$ und $R^3$ Chlor oder $R^1$ und $R^2$ Wasserstoff und $R^3$ Fluor, Chlor oder Methoxy bedeuten. Vorzugsweise bedeuten $R^3$ Chlor und $R^1$, $R^2$ und $R^4$ Wasserstoff.

$R^5$ bedeutet vorzugsweise Wasserstoff.

Wenn $R^6$ ein Rest der obigen Formel (A) ist, dann bedeutet $R^{12}$ zweckmässigerweise Wasserstoff, Methyl, Aethyl, n-Propyl, Isopropyl, n-Butyl, tert.-Butyl, n-Nonyl, 5-Nonyl, 3-Pyridylmethyl oder Carbamoylmethyl. Wenn $R^6$ ein Rest der obligen Formel (b) ist, dann bedeuten zweckmässigerweise entweder $R^{13}$ und $R^{14}$ beide Wasserstoff oder beide Aethyl oder zusammen mit dem Stickstoffatom 4-Methylpiperazin-1-

yl oder $R'^{13}$ Wasserstoff und $R'^{14}$ 4-Pyridyl, 2-Phenyläthyl, 2-Piperidinoäthyl, Carboxymethyl, Aethoxycarbonylmethyl, Carbamoylmethyl, 1-Aethoxycarbonyläthyl, 1,4-Bis-(äthoxycarbonyl)-2-butyl oder 2-(5-Chlor-2-pyridincarboxamido)äthyl. Vorzugsweise bedeuten $R^{12}$ Aethyl, n-Propyl, Isopropyl, n-Nonyl oder Carbamoylmethyl bzw. $R^{13}$ Wasserstoff und $R^{14}$ Wasserstoff, Aethoxycarbonylmethyl oder 1,4-Bis-(äthoxycarbonyl)-2-butyl.

$R^7$ bedeutet zweckmässigerweise Methyl, 3-Acetylaminopropyl, Amino oder p-Methoxyphenyl, vorzugsweise Methyl oder 3-Acetylaminopropyl.

$R^8$ und $R^9$ bedeuten zweckmässigerweise entweder beide Wasserstoff oder beide Methyl, vorzugsweise beide Wasserstoff.

$R^{10}$ und $R^{11}$ bedeuten vorzugsweise zusammen Oxo.

Ganz besonders bevorzugte Verbindungen der allgemeinen Formel I sind: N-[2-(4-Acetamidobutyryl)-5-chlorhydrocinnamoyl]glycinäthylester, 2-[4-(4-Acetamidobutyramido)butyryl]-5-chlorhydrozimtsäureäthylester, 2-[4-(4-Acetamidobutyramido)butyryl]-5-chlorhydrozimtsäureamid, 2-(4-Acetamidobutyryl)-5-chlorhydrozimtsäurenonylester, 2-(4-Acetamidobutyryl)-5-chlorhydrozimtsäureäthylester und 2-(4-Acetamidobutyryl)-N-(carbamoylmethyl)-5-chlorhydrozimtsäureamid.

Weitere besonders bevorzugte Verbindungen der allgemeinen Formel I sind: 2-[4-Acetamidobutyryl)-5-chlorhydrozimtsäureisopropylester, 2-[2-(4-Acetamidobutyryl)-5-chlorhydrocinnamoyl]-L-glutaminsäurediäthylester und 2-(4-Acetamidobutyryl)-5-chlorhydrozimtsäurecarbamoylmethylester.

Beispiele weiterer bevorzugter Verbindungen der allgemeinen Formel I sind: 2-(4-Acetamidobutyryl)-5-chlorhydrozimtsäureamid und 2-(4-Acetamidobutyryl)-5-chlorhydrozimtsäurepropylester.

Die Verbindungen der allgemeinen Formel I und pharmazeutisch annehmbare Salze davon können erfindungsgemäss dadurch hergestellt werden, dass man

a) ein Benzazecindion der allgemeinen Formel

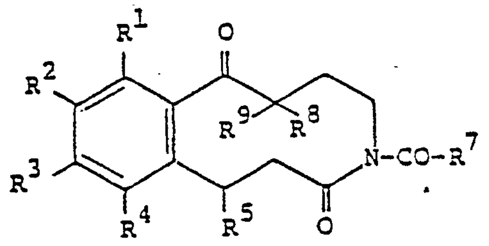

II

worin $R^1$, $R^2$, $R^3$, $R^4$, $R^5$, $R^7$, $R^8$ und $R^9$ obige Bedeutung besitzen, in Gegenwart einer Verbindung der allgemeinen Formel

$HOR^{12'}$ , III

worin $R^{12'}$ Wasserstoff oder $(C_1$-$C_{10})$-Alkyl bedeutet, mit einer Säure behandelt; oder

b) eine Verbindung der Formel I, worin $R^6$ einen Rest der Formel (a) und $R^{12}$ Wasserstoff bedeuten, oder ein reaktionsfähiges Derivat davon, zu einer entsprechenden Verbindung der Formel I, worin $R^6$ einen Rest der Formel (a) bedeutet und $R^{12}$ von Wasserstoff verschieden ist, verestert; oder

c) eine Verbindung der Formel I, worin $R^6$ einen Rest der Formel (a) und $R^{12}$ Wasserstoff bedeuten, oder ein reaktionsfähiges Derivat davon, mit einer Verbindung der allgemeinen Formel

$HNR^{13}R^{14'}$ IV

worin $R^{13}$ obige Bedeutung besitzt und $R^{14'}$ die oben für $R'^{14}$ angegebene Bedeutung besitzt, aber nicht Carboxy-$(C_1$-$C_4)$-alkyl ist,

umsetzt oder eine Verbindung der Formel I, worin $R^6$ einen Rest der Formel (b) und $R^{14}$ Carboxy-$(C_1$-$C_4)$-alkyl bedeuten, oder ein reaktionsfähiges Derivat davon, mit Ammoniak umsetzt; oder

d) eine Verbindung der Formel I, worin $R^{10}$ und $R^{11}$ zusammen Oxo bedeuten, reduziert; oder

e) eine Verbindung der Formel I, worin $R^6$ einen Rest der Formel (b) und $R^{14}$ $(C_1$-$C_4)$-Alkoxycarbonyl-$(C_1$-$C_4)$-alkyl bedeuten, zu einer entsprechenden Verbindung der Formel I, worin $R^6$ einen Rest der Formel (b) und $R^{14}$ Carboxy-$(C_1$-$C_4)$-alkyl bedeuten, hydrolysiert; oder

f) eine basische Verbindung der Formel I mittels einer Säure bzw. eine saure Verbindung der Formel I mittels einer Base in ein pharmazeutisch anwendbares Salz überführt.

Gemäss Variante a) des erfindungsgemässen Verfahrens erhält man Verbindungen der Formel I, worin $R^6$ einen Rest der obigen Formel (a), $R^{12}$ Wasserstoff oder $(C_1$-$C_{10})$-Alkyl und $R^{10}$ und $R^{11}$ zusammen Oxo

bedeuten.

Will man eine Verbindung der Formel II in eine ensprechende Verbindung der Formel I, worin $R^6$ einen Rest der Formel (a) und $R^{12}$ $(C_1-C_{10})$-Alkyl bedeuten, überführen, so verwendet man eine Verbindung der Formel III, worin $R^{12}$ $(C_1-C_{10})$-Alkyl bedeutet, d.h. einen entsprechenden Alkohol. Dieser Alkohol kann gleichzeitig als Lösungsmittel dienen; es ist aber auch möglich, ein anderes Lösungsmittel zuzufügen, beispielsweise einen halogenierten Kohlenwasserstoff, wie Methylenchlorid. Als Säure verwendet man zweckmässigerweise eine starke anorganische Säure, wie konzentrierte Salzsäure oder dergleichen. Die Reaktion erfolgt zweckmässigerweise bei etwa Raumtemperatur und dauert mehrere (z.B. 10) Stunden bis einige (z.B. 5) Tage.

Will man eine Verbindung der Formel II in eine entsprechende Verbindung der Formel I überführen, worin $R^6$ einen Rest der obigen Formel (a) und $R^{12}$ Wasserstoff bedeuten, so verwendet man eine Verbindung der Formel III, worin $R^{12}$ Wasserstoff bedeutet, d.h. Wasser. In diesem Fall geht man zweckmässigerweise so vor, dass man die Verbindung der Formel II in einem polaren aprotischen Lösungsmittel, wie Tetrahydrofuran, Acetonitril oder dgl., löst und dann wässrige Säure zugibt, beispielsweise verdünnte (z.B. 2N) Salzsäure oder dergleichen. Bezüglich der Reaktionstemperatur und der Reaktionsdauer verhält es sich analog wie vorstehend beschrieben.

Aspekt b) des erfindungsgemässen Verfahrens stellt eine Veresterung dar, welche nach allgemein üblichen und jedem Fachmann geläufigen Methoden durchgeführt werden kann.

Die zu veresternde Carbonsäure kann zweckmässigerweise in Form eines ihrer reaktionsfähigen Derivate eingesetzt werden, beispielsweise in Form eines Säurehalogenids (z.B. eines Säurechlorids), eines Imidazolids usw., welches dann mit der entsprechenden Hydroxylverbindung umgesetzt wird. Dabei braucht das reaktionsfähige funktionelle Derivat der Carbonsäure, welches beispielsweise mittels Thionylchlorid, Carbonyldiimidazol usw. hergestellt werden kann, nicht isoliert zu werden, sondern es kann in situ eingesetzt werden. Als reaktionsfähige funktionelle Derivate der zu veresternden Carbonsäuren können auch Silbersalze verwendet werden; aus einem derartigen Silbersalz erhält man den gewünschten Ester durch Umsetzung mit einem entsprechenden Halogenid. Die Reaktionsbedingungen, wie Temperatur, Dauer, Lösungsmittel usw. variieren je nach der Natur des eingesetzt reaktionsfähigen Carbonsäurederivats.

Freie Carbonsäuren kann man beispielsweise dann einsetzen, wenn man die Veresterung mittels einer eine olefinische Doppelbindung enthaltenden Komponente durchführt. So erhält man durch Behandlung einer freien Carbonsäure mit Isobutylen in Gegenwart einer geringen Menge einer starken Mineralsäure (z.B. konzentrierte Schwefelsäure) einen entsprechenden t-Butylester.

Gemäss Aspekt c) des erfindungsgemässen Verfahrens wird eine Carbonsäure in ein am Stickstoffatom gegebenenfalls entsprechend substuiertes Amid übergeführt, was nach an sich bekannten und jedem Fachmann geläufigen Methoden erfolgen kann.

Setzt man eine freie Carbonsäure ein, so erfolgt die Umsetzung mit einer Verbindung der Formel IV bzw. mit Ammoniak in Gegenwart eines wasserabspaltenden Mittels, wie Dicyclohexylcarbodiimid. Die Reaktion erfolgt zweckmässigerweise in einem unter den Reaktionsbedingungen inerten organischen Lösungsmittel, z.B. in einem Aether, wie Tetrahydrofuran usw., dauert mehrere (z.B. 10 - 20) Stunden und wird zweckmässigerweise bei etwa Raumtemperatur durchgeführt.

Als reaktionsfähige funktionelle Derivate der in diesem Aspekt des erfindungsgemässen Verfahrens eingesetzten Carbonsäuren eignen sich beispielsweise deren Ester, deren Imidazolide usw. wobei die Imidazolide nicht isoliert zu werden brauchen, sondern in situ eingesetzt werden können. Die Reaktionsbedingungen, wie Lösungsmittel, Temperatur, Dauer usw. variieren je nach der Natur des eingesetzten reaktionsfähigen funktionellen Carbonsäurederivats.

Gemäss Aspekt d) des erfindungsgemässen Verfahrens erhält man Verbindungen der Formel I, worin $R^{10}$ Wasserstoff und $R^{11}$ Hydroxy bedeuten. Die Reduktion erfolgt zweckmässigerweise mittels eines komplexen Hydrids in einem unter den Reaktionsbedingungen inerten organischen Lösungsmittel, beispielsweise mittels Natriumborhydrid in Methanol usw.. Die Reduktion erfolgt zweckmässigerweise bei etwa Raumtemperatur und dauert etwa 1 bis einige Stunden.

Die Hydrolyse gemäss Aspekt e) des erfindungsgemässen Verfahrens erfolgt nach an sich bekannten und jedem Fachmann geläufigen Methoden, zweckmässigerweise unter alkalischen Bedingungen, z.B. mittels eines Alkalimetallhydroxids, wie Natriumhydroxid, in Wasser oder in einem Gemisch von Wasser und einem mit Wasser mischbaren organischen Lösungsmittel, wie Tetrahydrofuran. Sie dauert etwa 1 bis einige Stunden und erfolgt zweckmässigerweise bei etwa Raumtemperatur.

Die Salzbildung gemäss Aspekt f) des erfindungsgemässen Verfahrens erfolgt nach allgemein üblichen und jedem Fachmann geläufigen Methoden. Basische Verbindungen der Formel I können in pharmazeutisch annehmbare Säureadditionssalze übergeführt werden, beispielsweise mit Chlorwasserstoff, Bromwasserstoff, Phosphorsäure, Schwefelsäure, Citronensäure, p-Toluolsulfonsäure und dergleichen. Saure Verbindungen der Formel I können mit geeigneten Basen pharmazeutisch annehmbare Salze bilden, beispielsweise Alkalimetallsalze, wie Natrium- oder Kaliumsalze, Erdalkalimetallsalze, wie Magnesium- oder Calciumsalze, sowie Salze mit Aminen, wie Triäthanolamin, Diäthylaminoäthanol, Triäthylamin, Trimethylamin, Diäthylamin usw..

Die Ausgangsprodukte der Formel II können dadurch hergestellt werden, dass man ein Benzochinolin-Derivat der allgemeinen Formel

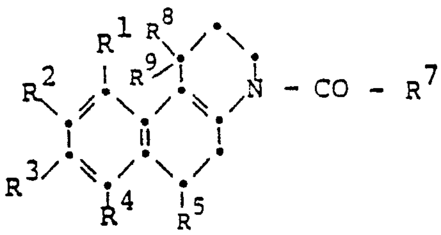

V

worin $R^1$, $R^2$, $R^3$, $R^4$, $R^5$, $R^7$, $R^8$ und $R^9$, die eingangs erwähnte Bedeutung besitzen, oxydiert.

Verbindungen der Formel V können ihrerseits dadurch erhalten werden, dass man

aa) ein Benzochinolinon-Derivat der allgemeinen Formel

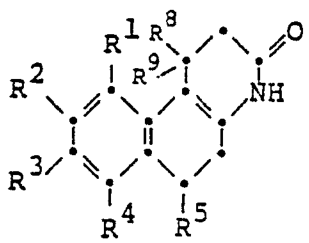

VI

worin $R^1$, $R^2$, $R^3$, $R^4$ $R^5$, $R^8$ und $R^9$ die eingangs erwähnte Bedeutung besitzen, reduziert oder

bb) eine Verbindung der Formel

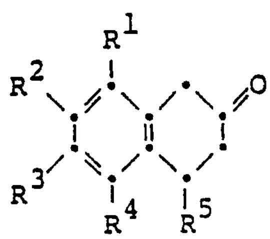

VII

worin $R^1$, $R^2$, $R^3$, $R^4$ und $R^5$ die eingangs erwähnte Bedeutung besitzen,
in Gegenwart einer starken Base mit einer Verbindung der allgemeinen Formel

$X\text{-}(CH_2)_3\text{-}NH_2$ VIII

worin X eine Abgangsgruppe bedeutet, umsetzt und die erhaltene Verbindung entsprechend N-substituiert.

Die Oxidation von Benzochinolin-Derivaten der allgemeinen Formel V zu entsprechenden Benzazecindionen der allgemeinen Formel II erfolgt zweckmässigerweise mittels m-Chlorperbenzoesäure in einem inerten organischen Lösungsmittel, beispielsweise in einem halogenierten Kohlenwasserstoff, wie Chloroform und dergleichen, zweckmässigerweise bei Temperaturen von etwa -20 °C bis etwa 30 °C, vorzugsweise zwischen etwa -5 °C und etwa Raumtemperatur.

Weiterhin kann diese Oxidation auch zweckmässigerweise mittels Kaliumpermanganat und Natriumperiodat durchgeführt werden, zweckmässigerweise in einem Zweiphasensystem, bestehend aus Wasser und einem damit nicht mischbaren organischen Lösungsmittel, z.B. einem halogenierten Kohlenwasserstoff, wie Methylenchlorid und dergleichen. Dabei wird vorteilhafterweie ein Phasentransferkatalysator beigefügt, insbesondere ein quartäres Ammoniumsalz, wie Benzyltriäthylammoniumchlorid und dergleichen. Auch die

Oxidation mittels Kaliumpermanganat/Natriumperiodat kann zweckmässigerweise bei Temperaturen zwischen etwa 0° C und etwa 30° C durchgeführt werden, beispielsweise bei etwa Raumtemperatur.

Für die Durchführung der fraglichen Oxidation eignen sich weiterhin Oxidationsmittel bzw. Oxidationssysteme, wie Peressigsäure, Wasserstoffperoxid und Ameisensäure oder p-Toluolsulfonsäure, Chromschwefelsäure, Jones-Reagenz und dergleichen.

Die Reduktion eines Benzochinolinon-Derivats der allgemeinen Formel VI, erfolgt zweckmässigerweise mittels eines komplexen Hydrids, wie Lithiumaluminiumhydrid und dergleichen, in einem unter den Reaktionsbedingungen inerten organischen Lösungsmittel, zweckmässigerweise in einem Aether, wie Tetrahydrofuran, Dioxan und dergleichen. Die Reduktion kann bei Temperaturen zwischen etwa Raumtemperatur und etwa 120° C erfolgen, zweckmässigerweise bei Rückflusstemperatur.

Die Umsetzung einer Verbindung der Formel VII mit einer Verbindung der Formel VIII erfolgt in Gegenwart einer starken Base, zweckmässigerweise in Gegenwart einer anorganischen Base, wie Kalium- oder Natriumhydroxid, einer quartären Ammoniumbase, wie Benzyltrimethylammoniumhydroxid u.dgl. Die in Formel VIII durch das Symbol X bezeichnete Abgangsgruppe ist zweckmässigerweise ein Halogenatom, insbesondere ein Chloratom, doch kommen auch äquivalente anderer Abgangsgruppen in Betracht, z.B. Alkylsulfonyloxygruppen, wie Mesyloxy, Arylsulfonyloxygruppen, wie Benzolsufonyloxy, p-Toluolsulfonyloxy, p-Brombenzolsulfonyloxy und dergleichen. Die Verbindung der Formel VIII wird zweckmässigerweise in Form eines Säureadditionssalzes eingesetzt, beispielsweise als Hydrochlorid. Die Reaktion erfolgt in Gegenwart eines unter den Reaktionsbedingungen inerten organischen Lösungsmittels, beispielsweise in einem aromatischen Kohlenwasserstoff, wie Toluol, und dergleichen. Die Umsetzung der Verbindungen der Formeln VII und VIII kann zweckmässigerweise zwischen Temperaturen von etwa 30° C und etwa 110° C erfolgen, vorzugsweise bei Rückflusstemperatur.

Sowohl bei der Reduktion eines Benzochinolinon-Derivates der allgemeinen Formel VI als auch bei der Umsetzung von Verbindungen der Formel VII und VIII erhält man am Stickstoff unsubstituierte Verbindungen, welche anschliessend N-acyliert werden müssen, und zur Herstellung von Verbindungend der Formel II, worin $R^7$ ($C_1$-$C_4$) -Alkyl oder ($C_1$-$C_4$)-Alkoxyphenyl bedeutet, verwendet man als Acylierungsmittel reaktionsfähige Derivate der entsprechenden Carbonsäuren, zweckmässigerweise Anhydride, wie Acetanhydrid, Carbonsäurechloride, wie p-Methoxybenzoylchlorid, und dergleichen. Zur Herstellung von Verbindungen der Formel II, worin $R^7$ Amino bedeutet, kann man die am Stickstoff unsubstituierte Verbindung mit $\alpha$-Chloracetylisocyanat und die erhaltene Verbindung mit Hydrazinhydrat umsetzten. Zur Herstellung von Verbindungen der Formel II, worin $R^7$ ($C_2$-$C_5$)-Alkanoylamino -($C_2$-$C_5$)-alkyl bedeutet, kann man die am Stickstoff unsubstituierte Verbindung mit einem Phthalimido-($C_2$-$C_5$)-alkanoylhalogenid umsetzen, das erhaltene Produkt mittels Hydrazinhydrat in das entsprechende freie Amin überführen und dieses schliesslich mit einem reaktionsfähigen Derivat der entsprechenden Carbonsärue, wie Acetylchlorid, acylieren.

Die Herstellung von Verbindungen der allgemeinen Formeln VI und VII erfolgt zweckmässigerweise gemäss dem nachfolgenden Reaktionsschema, worin $R^1$, $R^2$, $R^3$, $R^4$, $R^5$, $R^8$ und $R^9$ die eingangs erwähnte Bedeutung besitzen und $R^{12}$ und $R^{13}$ je niederes Alkyl oder zusammen mit dem Stickstoffatom einen heterocyclischen Rest bedeuten, wie Pyrrolin-1-yl, Pyrrolidin-1-yl, Piperidino, Morpholino, 4-($C_1$-$C_4$-Alkyl)-piperazin-1-yl und dergleichen.

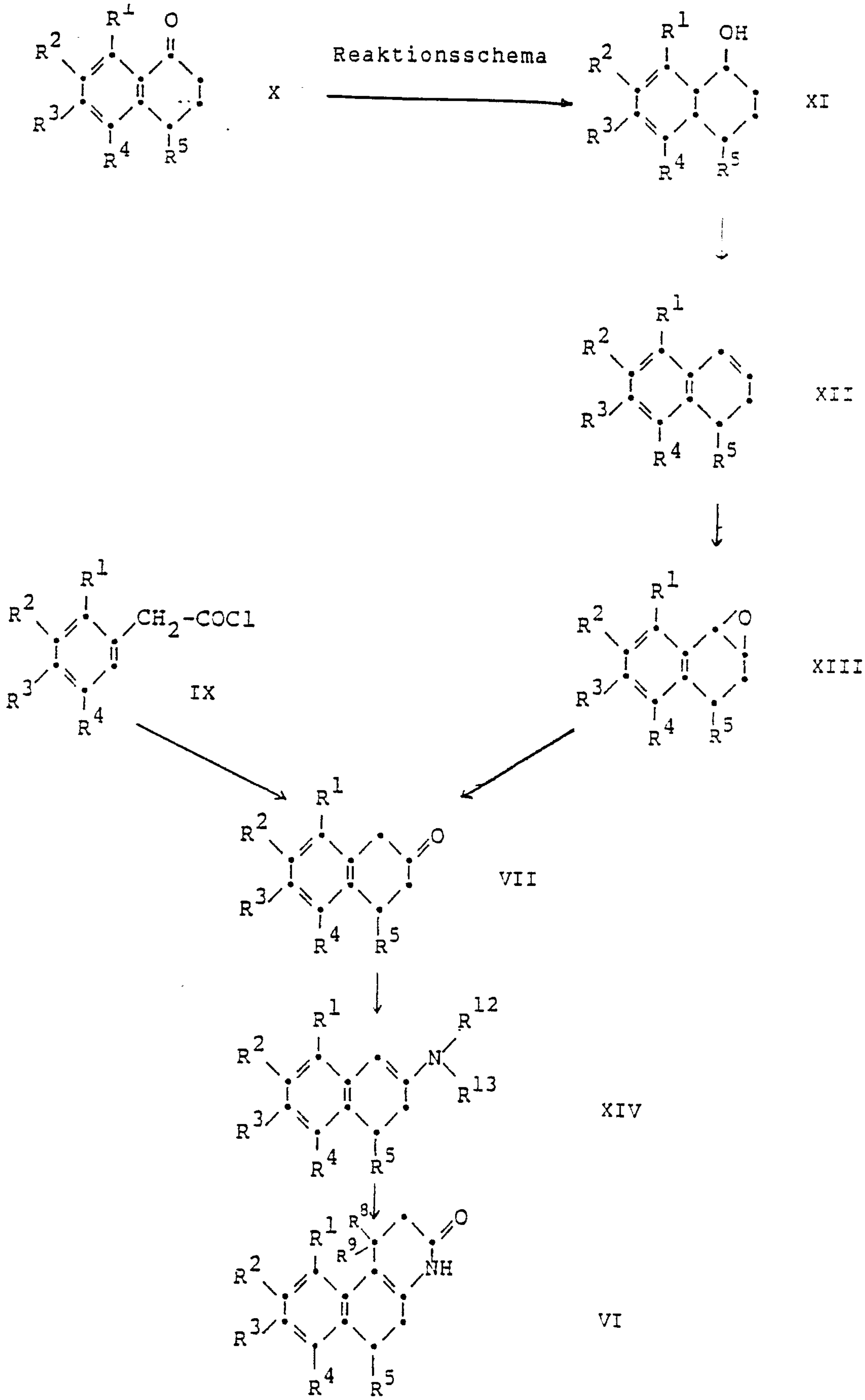


X

XI

XII

XIII

IX

VII

XIV

VI

Verbindungen der Formel VII können in einer Stufe aus Verbindungen der Formel IX hergestellt werden, und zwar durch Umsetzung mit Aethylen bzw. Styrol in Gegenwart von Aluminiumchlorid oder einer anderen für derartige Reaktionen als Katalysator geeigneten Lewis-Säure. Die Umsetzung erfolgt in Gegenwart eines unter den Reaktionsbedingungen inerten organischen Lösungsmittels, zweckmässigerweise in einem halogenierten Kohlenwasserstoff, wie Methylenchlorid.

Verbindungen der Formel VII können aber auch durch eine mehrstufige Synthese ausgehend von Verbindungen der Formel X hergestellt werden. Zunächst wird die Verbindung der Formel X zur entsprechenden Verbindung der Formel XI reduziert, zweckmässigerweise mit einem komplexen Hydrid, wie Natriumborhydrid oder dergleichen. Die erhaltene Verbindung der Formel XI wird dann zur entsprechenden Verbindung der Formel XII dehydratisiert, zweckmässigerweise unter sauren Bedingungen, z.B. mittels einer starken Säure, wie p-Toluolsulfonsäure oder dergleichen, in einem mit Wasser nicht mischbaren, jedoch azeotrop destillierenden Lösungsmittel bei Rückflusstemperatur, wobei das entstehende Wasser kontinuierlich entfernt wird. Die Verbindung der Formel XII wird dann zur entsprechenden Verbindung der Formel XIII oxydiert, zweckmässigerweise mittels m-Chlorperbenzoesäure oder dergleichen in einem unter den Reaktionsbedingungen inerten organischen Lösungsmittel, beispielsweise in einem chlorierten Kohlenwasserstoff, wie Methylenchlorid. Zu Verbindungen der Formel VII gelangt man dann ausgehend von entsprechenden Verbindungen der Formel XIII beispielsweise durch Behandlung mit einer ätherischen Lösung von Magnesiumbromid oder durch Behandlung mit einer organischen Sulfonsäure, wie p-Toluolsulfonsäure, o.dgl., in einem inerten organischen Lösungsmittel, wie Toluol o.dgl.

Zur Herstellung einer Verbindung der Formel XIV wird eine entsprechende Verbindung der Formel VII mit einem sekundären Amin der Formel $HNR^{12}R^{13}$ wie z.B. Pyrrolidin, umgesetzt, und zwar in Gegenwart einer Säure, zweckmässigerweise einer organischen Sulfonsäure, wie p-Toluolsulfonsäure oder dergleichen, in einem unter den Reaktionsbedingungen inerten organischen Lösungsmittel, beispielsweise in einem aromatischen Kohlenwasserstoff, wie Benzol; das hierbei entstehende Wasser wird aus dem Reaktionssystem entfernt, beispielsweise durch Zugabe von Molekularsieb oder durch azeotrope Destillation. Zu Verbindungen der Formel VI gelangt man schliesslich dadurch, dass man eine entsprechende Verbindung der Formel XIV mit Acrylamid, 3,3-Dimethylacrylamid o. dgl zur Reaktion bringt. Die Umsetzung mit Acrylamid erfolgt zweckmässigerweise in Gegenwart einer Säure, z.B. einer organischen Sulfonsäure, wie p-Toluolsulfonsäure, eines sauren Ionenaustauschers oder dergleichen, bei Temperaturen von etwa 100° C bis etwa 200° C, zweckmässigerweise von etwa 100-150° C, wobei als Lösungsmittel ($C_1$-$C_4$)-Alkanole, wie Aethanol, oder dergleichen verwendet werden können. Die Umsetzung mit 3,3-Dimethylacrylamid erfolgt zweckmässigerweise in Gegenwart von tetramethoxysilan und Caesiumfluorid in einem aromatischen Kohlenwasserstoff, wie Toluol, bei etwa Rückflusstemperatur.

Wie eingangs erwähnt, sind die Hydrozimtsäurederivate der Formel I und deren pharmazeutisch anwendbare Salze neue Verbindungen mit äusserst wertvollen pharmakodynamischen Eigenschaften. Sie weisen nur eine geringe Toxizität auf, und es hat sich gezeigt, dass sie in dem nachstehend beschriebenen Tierversuch experimentell hervorgerufener cerebraler Insuffizienz entgegenzuwirken vermögen.

Die Test-Apparatur ist eine "Skinner box" mit einem elektrifizierbaren Gitterboden (30 x 40 cm) und einer grauen Plastik-Plattform (15 x 15 x 0,8 cm) in der Ecke vorne rechts. Unerfahrene männliche Ratten (100-120 g) werden einzeln auf die Plattform gebracht. Sobald sie auf den Gitterboden hinabsteigen, erhalten sie einen elektrischen Fuss-Schock (0,8 mA). Die normale Reaktion unerfahrener Ratten ist, daraufhin auf die Plattform zurückzuspringen. Da jedoch die Ratten immer nochmals herabzuklettern versuchen, muss die Fuss-Schock-Prozedur für jedes Tier drei- bis fünfmal wiederholt werden. Nach diesen drei bis fünf Wiederholungen pro Tier haben die Ratten eine sogenannte "passive avoidance response" erlernt, d.h. sie versuchen nicht mehr, auf den Gitterboden hinabzusteigen, weil sie wissen, dass sie bestraft werden.

Unmittelbar anschliessend werden drei Gruppen von je 30 Tieren gebildet. Die erste Gruppe erhält eine Injektion (i.p.) von 0,3 mg/kg Scopolamin sowie destilliertes Wasser (2 ml/kg p.o.). Die zweite Gruppe erhält eine Injektion (i.p.) von 0,3 mg/kg Scopolamin und eine orale Dosis der Testsubstanz. Die dritte Gruppe erhält ausschliesslich destilliertes Wasser (p.o.).

2 Stunden später wird jede Ratte einmal auf die Plattform in der "Skinner box" gesetzt. Das Kriterium für die Beurteilung dieses Tests zur Ermittlung einer Präparatwirkung auf das Kurzzeit-Gedächtnis ist, ob das Tier während 60 Sekunden auf der Plattform bleibt oder nicht (das Ergebnis kann also für jedes Tier nur "ja" oder "nein" lauten). Die statistische Signifikanz der Unterschiede zwischen den bei der ersten und der zweiten Gruppe erhaltenen Resultaten wird mittels des Chi-Quadrat-Tests ermittelt.

70 - 75% der lediglich mit destilliertem Wasser (p.o.) behandelten Tiere erinnern sich 2-4 Stunden nach Erlernen der "passive avoidance response" noch daran, dass sie auf der Plattform bleiben sollten. Bei 85-92% der mit Scopolamin (0,3 mg/kg i.p.) und destilliertem Wasser (p.o.) behandelten Tiere lässt sich während 3-4 Stunden ein retrograder Effekt auf das Kurzzeitgedächtnis feststellen, d.h. sie haben vergessen, dass sie auf der Plattform bleiben müssen. Eine Substanz, welche cerebraler Insuffizienz entgegenzuwirken vermag, kann die durch die Injektion (i.p.) von 0,3 mg/kg Scopolamin hervorgerufene Blockierung des Kurzzeitgedächtnisses aufheben. Eine Dosis eines Präparats wird dann als aktiv gegen Scopolamin bezeichnet, wenn die Anzahl der positiven Ergebnisse ("ja") von derjenigen mit Scopolamin (0,3 mg/kg i.p.) und nur destilliertem Wasser (p.o.) behandelter Kontroll-Tiere signifikant verschieden ist.

In der nachstehenden Tabelle ist angegeben, bei welchen Dosen bestimmte Verbindungen der Formel I in dem vorstehend beschriebenen Versuch eine signifikante Aktivität zeigen. Die Tabelle enthält ausserdem Angaben über die akute Toxizität ($DL_{50}$ in mg/kg bei einmaliger oraler Verabreichung an Mäuse).

| $R^1$ | $R^2$ | $R^3$ | $R^4$ | $R^5$ | $R^6$ | $R^7$ | $R^8$ | $R^9$ | $R^{10}$ | $R^{11}$ | Signifikant wirksame Dosen mg/kg p.o. | DL 50 mg/kg p.o. |
|---|---|---|---|---|---|---|---|---|---|---|---|---|
| H | H | Cl | H | H | $NHCH_2COOC_2H_5$ | $CH_3$ | H | H | Oxo | | 0,003<br>0,03<br>0,3<br>3<br>10 | > 5000 |
| H | H | Cl | H | H | $OC_2H_5$ | $(CH_2)_3NHCOCH_3$ | H | H | Oxo | | 0,003<br>0,03<br>0,3 | > 5000 |
| H | H | Cl | H | H | $NH_2$ | $(CH_2)_3NHCOCH_3$ | H | H | Oxo | | 0,00001<br>0,00003<br>0,0003<br>0,003<br>0,03 | |
| H | H | Cl | H | H | $OCH(CH_3)_2$ | $CH_3$ | H | H | Oxo | | 0,01<br>0,03<br>0,1<br>0,3<br>1<br>3<br>10<br>30 | > 4000 |
| H | H | Cl | H | H | $OC_2H_5$ | $CH_3$ | H | H | Oxo | | 0,03<br>0,1<br>0,3<br>1<br>3 | > 5000 |
| H | H | Cl | H | H | $NH-CH(-(CH_2)-COOC_2H_5)-COOC_2H_5$ | $CH_3$ | H | H | Oxo | | 0,1<br>0,3<br>3 | > 5000 |
| H | H | Cl | H | H | $OCH_2CONH_2$ | $CH_3$ | H | H | Oxo | | 0,03<br>0,3<br>1 | |

Tabelle (Forts.)

| $R^1$ | $R^2$ | $R^3$ | $R^4$ | $R^5$ | $R^6$ | $R^7$ | $R^8$ | $R^9$ | $R^{10}$ | $R^{11}$ | Signifikant wirksame Dosen mg/kg p.o. | DL 50 mg/kg p.o. |
|---|---|---|---|---|---|---|---|---|---|---|---|---|
| H | H | Cl | H | H | $NH_2$ | $CH_3$ | H | H | Oxo | | 0,03<br>0,3 | > 4000 |
| H | H | Cl | H | H | $O-(CH_2)_2-CH_3$ | $CH_3$ | H | H | Oxo | | 0,1<br>0,3 | > 5000 |
| H | H | Cl | H | H | $O-(CH_2)_8-CH_3$ | $CH_3$ | H | H | Oxo | | 0,03<br>0,1<br>0,3<br>3 | > 5000 |

Die Verbindungen der Formel I und deren pharmazeutisch anwendbare Salze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Wie weiter vorne erwähnt, sind Arzneimittel, enthaltend eine Verbindung der Formel I oder ein pharmazeutisch anwendbares Salz davon ebenfalls Gegenstand der vorliegenden Erfindung, weiterhin auch ein Verfahren zur Herstellung derartiger Arzneimittel, welches dadurch gekennzeichnet ist, dass man eine oder mehrere Verbindungen der Formel I und/oder pharmazeutisch anwendbare Salze davon und erwünschtenfalls einen oder mehrere andere therapeutisch wirksame Stoffe zusammen mit einem oder mehreren therapeutisch inerten Exzipientien in eine galenische Darreichungsform bringt.

Zur Herstellung von Tabletten, Lacktabletten, Dragées und Hartgelatinekapseln kann man als Excipientien z.B. Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose und dergleichen.

Für Injektionslösungen eignen sich als Excipientien z.B. Wasser, Alkohole, Polyole, Glyzerin, vegetabile Oele etc.

Für Suppositorien eignen sich als Excipientien z.B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Fàrbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der Formel I und pharmazeutisch anwendbare Salze davon bei der Bekämpfung bzw. Verhütung der cerebralen Insuffizienz bzw. bei der Verbesserung cognitiver Funktionen (wie Erinnerungsvermögen, Lernfähigkeit, Interesse an der Umwelt und Selbstsorge) verwenden, beispielswiese in der Geriatrie, bei Intoxikationen, wie Alkoholismus, und bei cerebro-vaskulären Störungen; mögliche weitere Einsatzgebiete sind vestibuläre Störungen (wie Menière-Krankheit) und Entwicklungsstörungen (wie Dyslexie). Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 1 bis 2500 mg angemessen sein, wobei aber die soeben angegebene obere Grenze auch überschritten werden kann, wenn sich dies als angezeigt erweisen sollte.

Gegenstand der Erfindung ist schliesslich auch die Verwendung der Verbindungen der Formel I und von pharmazeutisch anwendbaren Salzen davon zur Herstellung von Arzneimitteln für die Bekämpfung bzw. Verhütung der cerebralen Insuffizienz bzw. für die Verbesserung cognitiver Funktionen.

In den nachfolgenden Beispielen, welche die vorliegende Erfindung illustrieren, deren Umfang jedoch in keiner Weise einschränken sollen, sind sämtliche Temperaturen in Celsiusgraden angegeben.

## Beispiel 1

a) Man löst 1155 g (6.4 Mol) 6-Chlor-3,4-dihydro-2(1H)-naphthalinon in 5 l Toluol, tropft 500 g (7.0 Mol) Pyrrolidin und anschliessend eine Lösung von 26 g (0.14 Mol) p-Toluolsulfonsäure-Monohydrat in Toluol zu und kocht unter Rückfluss am Wasserabscheider. Wenn ca. 120 ml Wasser abgeschieden sind, destilliert man 4 l Toluol ab und lässt langsam abkühlen. Dabei kristallisiert ein Festkörper aus. Filtrieren und Waschen mit Aceton ergibt 1-(6-Chlor-3,4-dihydro-2-naphthyl)pyrrolidin mit Schmelzpunkt 117-118°. Einengen der Mutterlauge. Suspendieren des Rückstandes in Aether, Filtieren und Waschen mit Aceton ergibt eine weitere Portion des obigen Produkts mit Schmelzpunkt 117-118°.

b) Man kocht 701 g (3 Mol) 1-(6-Chlor-3,4-dihydro-2-naphthyl)pyrrolidin und 640 g (19 Mol) Acrylamid in 7 l Aethanol unter Zusatz von 70 g Amberlit IR200 3 Tage lang unter Rückfluss, filtriert den ausgefallenen Feststoff ab, kristallisiert extraktiv mit Dioxan und erhält 8-Chlor-1,4,5,6-tetrahydrobenzo[f]chinolin-3(2H)-on mit Schmelzpunkt 228-230°.

c) Man suspendiert unter Argon 147 g (1.94 Mol) Lithiumaluminiumhydrid in 4 l Tetrahydrofuran, gibt langsam 454 g (1.94 Mol) 8-Chlor-1,4,5,6-tetrahydrobenzo[f]chinolin-3(2H)-on zu und kocht 2.5 Stunden unter Rückfluss. Dann kühlt man ab. tropft 470 ml 18-prozentige Natronlauge zu, rührt 30 Minuten bei Raumtemperatur, filtriert und wäscht den Filterrückstand mit Tetrahydrofuran. Beim Eindampfen des Filtrats erhält man 8-Chlor-1,2,3,4.5,6-hexahydrobenzo[f]chinolin als gelbes Oel.

d) Man löst 229 g (1.04 Mol) 8-Chlor-1,2,3,4,5,6-hexahydrobenzo[f]chinolin in 2 l Methylenchlorid, versetzt mit 115 g (1.14 Mol) Triäthylamin und tropft bei 0° 89.5 g (1.14 Mol) Acetylchlorid in 400 ml Methylenchlorid zu. Nach 1-stündigem Rühren bei Raumtemperatur giesst man das Gemisch in Wasser, extrahiert mit Methylenchlorid und trocknet die Methylenchloridphase mit Magnesiumsulfat. Abdestillieren des Lösungsmittels im Vakuum ergibt ein Rohprodukt, das man in 500 ml Aether suspendiert und abfiltriert. Man erhält 4-Acetyl-8-chlor-1,2,3,4,5,6-hexahydrobenzo[f]chinolin mit Schmelzpunkt 106-108°. Einengen der Mutterlauge und Chromatographieren (Kieselgel/Chloroform) ergibt eine weitere Portion mit Schmelzpunkt 106-108°.

4-Acetyl-8-chlor-1,2,3,4,5,6-hexahydrobenzo[f]chinolin kann aus 6-Chlor-3,4-dihydro-2(1H)-naphthalinon auch wie folgt hergestellt werden:

Unter Argon löst man 100 g 6-Chlor-3,4-dihydro-2(1H)-naphthalinon (0.55 Mol) in 2 l Toluol, versetzt mit 64.0 g pulverisiertem Kaliumhydroxid, erhitzt auf Siedetemperatur, gibt während 30 Minuten portionsweise 165 g 3-Chlorpropylaminhydrochlorid zu und kocht am Wasserabscheider bis im Dünnschichtchromatogramm kein Edukt mehr nachzuweisen ist. Nach Abkühlen auf Raumtemperatur versetzt man mit 155 ml Triäthylamin. Unter Eiskühlung, so dass eine Innentemperatur von 25° nicht überschritten wird, versetzt man tropfenweise mit 60 ml Acetylchlorid, gelöst in 450 ml Toluol. Man rührt 1 Stunde bei Raumtemperatur, extrahiert mit Wasser/Methylenchlorid, trocknet mit Magnesiumsulfat, destilliert das Lösungsmittel im Vakuum ab und erhält 4-Acetyl-8-chlor-1,2,3,4,5,6-hexahydrobenzo[f]chinolin in Form brauner Kristalle, die als Rohprodukt für die nachstehend beschriebene Reaktion eingesetzt werden können.

e) Man löst 115 g (0.44 Mol) 4-Acetyl-8-chlor-1,2,3,4,5,6-hexahydrobenzo[f]chinolin in 1 l Methylenchlorid und tropft bei 0° eine Suspension von 189 g (0.93 Mol) m-Chlorperbenzoesäure (85-prozentig) in 500 ml Methylenchlorid zu. Nach 1-stündigem Rühren bei Raumtemperatur filtriert man vom gebildeten Niederschlag ab und extrahiert mit 2N Natronlauge und mit Wasser. Trocknen der organischen Phase mit Magnesiumsulfat, Abdestillieren des Lösungsmittels im Vakuum und Umkristallisieren des Rückstands aus Essigester-Aether ergibt 4-Acetyl-11-chlor-1,2,4,5,6,7-hexahydro-4-benzazecin-3,8-dion als weisse Kristalle mit Schmelzpunkt 154-156°.

f) Zu einer Lösung von 4.00 g (0.014 Mol) 4-Acetyl-11-chlor-1,2,4,5,6,7-hexahydro-4-benzazecin-4,8-dion in 200 ml Methanol gibt man 1 ml konzentrierte Salzsäure und rührt 20 Stunden bei Raumtemperatur. Man engt die Lösung ein, extrahiert mit Methylenchlorid/Wasser, trocknet mit Magnesiumsulfat und destilliert das Lösungsmittel im Vakuum ab. Chromatographieren an Kieselgel mit Essigester und dann an Aluminiumoxid mit Essigester und Kristallisieren aus t-Butyl-methyläther ergibt 2-(4-Acetamidobutyryl)-5-chlorhydrozimtsäuremethylester als weisse Kristalle mit Schmelzpunkt 46-48°.

## Beispiel 2

Zu einer Lösung von 4.00 g (0.014 Mol) 4-Acetyl-11-chlor-1,2,4,5,6,7-hexahydro-4-benzazecon-3,8-dion in 250 ml Aethanol gibt man 5 ml konzentrierte Salzsäure und rührt 30 Stunden bei Raumtemperatur. Man engt die Lösung ein, extrahiert mit Methylenchlorid/Wasser, trocknet mit Magnesiumsulfat und destilliert das Lösungsmittel im Vakuum ab. Chromatographieren an Kieselgel mit Essigester-Hexan (2:1) und Kristallisieren aus Aether ergibt 2-(4-Acetamidobutyryl)-5-chlor-hydrozimtsäureäthylester als beige Kristalle mit Schmelzpunkt 64-66°.

## Beispiel 3

Zu einer Lösung von 7.35 g (0.025 Mol) 4-Acetyl-11-chlor-1,2,4,5,6,7-hexahydro-4-benzazecin-3,8-dion in 400 ml n-Propanol gibt man 10 ml konzentrierte Salzsäure und rührt 24 Stunden bei Raumtemperatur.

Man engt die Lösung ein, extrahiert mit Methylenchlorid/Wasser, trocknet mit Magnesiumsulfat und destilliert das Lösungsmittel im Vakuum ab. Chromatographieren an Kieselgel mit Essigester-Hexan (2:1) und Kristallisieren aus Essigester-Hexan ergibt 2-(4-Acetamidobutyryl)-5-chlorhydrozimtsäurepropylester als weisse Kristalle mit Schmelzpunkt 60-62°.

Beispiel 4

Zu einer Lösung von 4.00 g (0.014 Mol) 4-Acetyl-11-chlor-1,2,4,5,6,7-hexahydro-4-benzazecin-3,8-dion in 250 ml Isopropanol gibt man 5 ml konzentrierte Salzsäure und rührt 24 Stunden bei Raumtemperatur. Man engt die Lösung ein, extrahiert mit Methylenchlorid/Wasser, trocknet mit Magnesiumsulfat und destilliert das Lösungmittel im Vakuum ab. Chromatographieren an Kieselgel mit Essigester-Hexan (2:1) und Kristallisieren aus Aether ergibt 2-(4-Acetamidobutyryl)-5-chlorhydrozimtsäureisopropylester als weisse Kristalle mit Schmelzpunkt 35-37°.

Beispiel 5

Zu einer Lösung von 10.00 g (0.034 Mol) 4-Acetyl-11-chlor-1,2,4,5,6,7-hexahydro-4-benzazecin-3,8-dion in 70 ml n-Butanol und 50 ml Methylenchlorid gibt man 15 ml konzentrierte Salzsäure und rührt 18 Stunden bei Raumtemperatur. Man engt die Lösung ein, extrahiert mit Methylenchlorid/Wasser, trocknet mit Magnesiumsulfat und destilliert das Lösungmittel im Vakuum ab. Chromatographieren an Kieselgel mit Essigester und Kristallisieren aus Aether ergibt 2-(4-Acetamidobutyryl)-5-chlorhydrozimtsäurebutylester als weisse Kristalle mit Schmelzpunkt 56-57°.

Beispiel 6

Zu einer Lösung von 10.00 g (0.034 Mol) 4-Acetyl-11-chlor-1,2,4,5,6,7-hexahydro-4-benzazecin-3,8-dion in 90 ml n-Nonanol und 50 ml Methylenchlorid gibt man 15 ml konzentrierte Salzsäure und rührt 15 Stunden bei Raumtemperatur. Man engt die Lösung ein, extrahiert mit Methylenchlorid/Wasser, trocknet mit Magnesiumsulfat und destilliert das Lösungmittel im Vakuum ab. Chromatographieren an Kieselgel mit Essigester-Hexan (4:1) und Kristallisieren aus Aether ergibt 2-(4-Acetamidobutyryl)-5-chlorhydrozimtsäurenonylester als weisse Kristalle mit Schmelzpunkt 55-57°.

Beispiel 7

Zu einer Lösung von 10.00 g (0.034 Mol) 4-Acetyl-11-chlor-1,2,4,5,6,7-hexahydro-4-benzazecin-3,8-dion in 90 ml 5-Nonanol und 50 ml Methylenchlorid gibt man 15 ml konzentrierte Salzsäure und rührt 18 Stunden bei Raumtemperatur. Man engt die Lösung ein, extrahiert mit Methylenchlorid/Wasser, trocknet mit Magnesiumsulfat und destilliert das Lösungmittel im Vakuum ab. Chromatographieren an Kieselgel mit Essigester ergibt 2-(4-Acetamidobutyryl)-5-chlorhydrozimtsäure-1-butylpentylester als gelbes Oel.

Beispiel 8

a) Zu einer Lösung von 36.0 g (0.143 Mol) 4-Phthalimidobuttersäurechlorid in 360 ml Methylenchlorid gibt man bei 0° 31.4 g (0.143 Mol) 8-Chlor-1,2,3,4,5,6-hexahydrobenzo[f]chinolin, gelöst in 300 ml Methylenchlorid, und rührt 1 Stunde bei Raumtemperatur. Man engt die Lösung ein, extrahiert mit Methylenchlorid und wässriger Natriumhydrogencarbonat-Lösung, trocknet mit Magnesiumsulfat und destilliert das Lösungmittel im Vakuum ab. Extraktives Kristallisieren mit Aceton ergibt 8-Chlor-1,2,3,4,5,6-hexahydro-4-(4-phthalimidobutyryl)benzo[f]chinolin als weisse Kristalle mit Schmelzpunkt 182-183°.

b) Man kocht eine Suspension von 10.9 g (0.025 Mol) 8-Chlor-1,2,3,4,5,6-hexahydro-4-(4-phthalimidobutyryl)benzo[f]chinolin und 3.2 ml (0.065 Mol) Hydrazinhydrat in 150 ml Aethanol 2 Stunden bei Rückflusstemperatur, engt die Lösung ein, extrahiert mit Methylenchlorid/Wasser, trocknet mit Natrium- sulfat und destilliert das Lösungsmittel im Vakuum ab. Man löst in 10 ml 5.5 N methanolischer Salzsäure und fällt mit Aether. Umkristallisieren aus Methanol/Aether ergibt 4-(4-Aminobutyryl)-8-chlor-1,2,3,4,5,6-hexahydrobenzo-[f]chinolin-hydrochlorid als beige Kristalle mit Schmelzpunkt 225°.

c) Zu einer Suspension von 5.09 g (0.015 Mol) 4-(4-Aminobutyryl)-8-chlor-1,2,3,4,5,6-hexahydrobenzo[f]chinolin in 50 ml Methylenchlorid gibt man 5 ml (0.036 Mol) Triäthylamin, fügt dann bei 0° 1.33 ml (0.019 Mol) Acetylchlorid, gelöst in 15 ml Methylenchlorid, zu und rührt 1 Stunde bei Raumtemperatur. Man extrahiert mit Methylenchlorid/Wasser, trocknet mit Magnesiumsulfat und destilliert das Lösungsmittel im Vakuum ab. Kristallisieren aus Methylenchlorid/Aether, Chromatographieren an Kieselgel mit Essigester/Methanol (9:1) und erneutes Kristallisieren aus Methylenchlorid/Aether ergibt N-[4-[8-Chlor-2,3,5,6-tetrahydrobenzo[f]chinolin-4(1H)-yl]-4-oxobutyl]acetamid als weisse Kristalle mit Schmelzpunkt 142- 143°.

d) Zu einer Lösung von 4.00 g (0.016 Mol) N-[4-[8-Chlor-2,3,5,6-tetrahydrobenzo[f]chinolin-4(1H)-yl]-4-oxobutyl]acetamid in 40 ml Methylenchlorid gibt man bei 0° 4.80g (0.024 Mol) 85-prozentige m-Chlorperbenzoesäure, gelöst in 50 ml Methylenchlorid, und rührt 1 Stunde bei Raumtemperatur. Man extrahiert mit Methylenchlorid/Wasser, trocknet mit Magnesiumsulfat und destilliert das Lösungsmittel im Vakuum ab. Chromatographieren an Kieselgel mit Essigester/Methanol (9:1) und Kristallisieren aus Essigester/Hexan ergibt N-[3-[(11-Chlor-2,3,5,6,7,8-hexahydro-3,8-dioxo-4-benzazecin-4-(1H)-yl)carbonyl]-propyl]acetamid als weisse Kristalle mit Schmelzpunkt 135-136°.

e) Zu einer Lösung von 2.66 g (0.007 Mol) N-[3-[(11-Chlor-2,3,5,6,7,8-hexahydro-3,8-dioxo-4-benzazecin-4(1H)-yl)carbo nyl]propyl]acetamid in 100 ml Aethanol und 30 ml Methylenchlorid gibt man 1 ml konzentrierte Salzsäure und rührt 5 Tage bei Raumtemperatur. Man engt die Lösung ein, extrahiert mit Methylenchlorid/Wasser, trocknet mit Magnesiumsulfat und destilliert das Lösungsmittel im Vakuum ab. Chromatographieren an Aluminiumoxid mit Essigester/Aethanol (19:1) und Kristallisieren aus Methylenchlorid/Aether ergibt 2-[4-(4-Acetamidobutyramido)butyryl]-5-chlorhydrozimtsäureäthylester als weisse Kristalle mit Schmelzpunkt 95°.

## Beispiel 9

a) Man löst 15.0 g (0.06 Mol) 8-Chlor-1,2,3,4,5,6-hexahydrobenzo[f]chinolin in 150 ml Methylenchlorid, versetzt mit 6.68 g (0.066 Mol) Triäthylamin und tropft bei 0° 11.2 g (0.066 Mol) p-Methoxybenzoylchlorid in 50 ml Methylenchlorid zu. Nach 1-stündigem Rühren bei Raumtemperatur extrahiert man mit Wasser, trocknet mit Magnesiumsulfat und destilliert das Lösungsmittel im Vakuum ab. Kristallisieren (Methylenchlorid-Aether) ergibt 4-(p-Methoxybenzoyl)-8-chlor-1,2,3,4,5,6-hexahydrobenzo[f]chinolin als weisse Kristalle mit Schmelzpunkt 182-183°.

b) Man löst 8.80 g (0.025 Mol) 4-(p-Methoxybenzoyl)-8-chlor-1,2,3,4,5,6-hexahydrobenzo[f]chinolin in 100 ml Chloroform und tropft bei 0° eine Suspension von 10.3 g (0.051 Mol) 85-prozentige m-Chlorperbenzoesäure in 100 ml Chloroform zu. Nach 1-stündigem Rühren bei Raumtemperatur extrahiert man mit 2N Natronlauge und Wasser, trocknet mit Magnesiumsulfat und destilliert das Lösungsmittel im Vakuum ab. Chromatographieren (Kieselgel, Aether-Hexan, 2:1) und Kristallisieren (Aether-Hexan) ergibt 4-(p-Methoxybenzoyl)-11-chlor-1,2,4,5,6,7-hexahydro-4-benzazecin-3,8-dion als weisse Kristalle mit Schmelzpunkt 143°.

c) Zu einer Lösung von 3.60 g (0.009 Mol) 4-(p-Methoxybenzoyl)-11-chlor-1,2,4,5,6,7-hexahydro-4-benzazecin-3,8-dion in 200 ml Methanol und 50 ml Methylenchlorid gibt man 5 ml konzentrierte Salzsäure und rührt 60 Stunden bei Raumtemperatur. Man engt die Lösung ein, extrahiert mit Methylenchlorid/Wasser, trocknet mit Magnesiumsulfat und destilliert das Lösungsmittel im Vakuum ab. Chromatographieren an Kieselgel mit Hexan-Essigester (1:1) und Kristallisieren aus Methylenchlorid/Hexan ergibt 2-[4-(p-Methoxybenzoyl)amidobutyryl]-5-chlorhydrozimtsäuremethylester als weisse Kristalle mit Schmelzpunkt 111°.

Beispiel 10

Zu einer Lösung von 103 g (0.35 Mol) 4-Acetyl-11-chlor-1,2,4,5,6,7-hexahydro-4-benzazecin-3,8-dion in 1 1 Tetrahydrofuran gibt man 500 ml 2N Salzsäure und rührt über Nacht bei Raumtemperatur. Man engt im Vakuum ein, versetzt mit Methylenchlorid und extrahiert zwei mal mit 2N Natronlauge; dann säuert man die Wasserphase mit 6N Salzsäure an und extrahiert mit Methylenchlorid. Abdestillieren des Lösungsmittels im Vakuum und Chromatographieren an Kieselgel mit Methylenchlorid/Methanol (20:1) liefert 2-(4-Acetamidobutyryl)-5-chlorhydrozimtsäure mit Schmelzpunkt 90-92°.

Beispiel 11

a) Zu 218 g Aluminiumchlorid in 1000 ml Methylenchlorid werden unter Rühren zwischen 0° und 5° innerhalb von 1 Stunde 154.8 g 2-Chlor-phenylacetylchlorid, gelöst in 290 ml Methylenchlorid, zugetropft. Danach leitet man während 40 Minuten bei 0° bis 5° Aethylen ein. Man rührt 1 Stunde bei Raumtemperatur und versetzt zwischen 0° und 5° mit 570 ml Wasser. Die Methylenchloridphase wäscht man mit 2 x 500 ml 2N Salzsäure, 2 x 500 ml Natriumhydrogencarbonatlösung und 700 ml Wasser, trocknet mit Natriumsulfat und engt im Vakuum ein. Das aus 400 ml tiefsiedendem Petroläther kristallisierte 8-Chlor-3,4-dihydro-2(1H)-naphthalinon zeigt einen Schmelzpunkt von 56-59°.

b) 70.0 g 8-Chlor-3,4-dihydro-2(1H)-naphthalinon werden in 550 ml Benzol und 33 ml Pyrrolidin in Gegenwart von 1.4 g p-Toluolsulfonsäure 2,5 Stunden am Rückfluss gekocht. Das erhaltene rohe 1-(8-Chlor-3,4-dihydro-2-naphthyl)-pyrrolidin wird ohne Reinigung weiterverarbeitet.

c) Zu 89.4 g rohem 1-(8-Chlor-3,4-dihydro-2-naphthyl)pyrrolidin werden 56.0 g Acrylamid und 3.0 g wasserfreie p-Toluolsulfonsäure zugegeben. Man erhitzt unter Stickstoff je 2 Stunden bei 100° und bei 150°, extrahiert mit Methylenchlorid-Wasser und filtriert durch 500 g Kieselgel und destilliert das Lösungsmittel im Vakuum ab. Das erhaltene 10-Chlor-1,4,5,6-tetrahydrobenzo[f]chinolin-3(2H)-on zeigt nach Umkristallisieren aus Essigester einen Schmelzpunkt von 186-187°.

d) Zu einer gerührten Suspension von 6.49 g Lithiumaluminiumhydrid in 240 ml trockenem Tetrahydrofuran werden unter Stickstoff innerhalb von 35 Minuten bei 20° bis 25° portionenweise 20.0 g 10-Chlor-1,4,5,6-tetrahydrobezno[f]chinolin-3(2H)-on zugegeben. Das Reaktionsgemisch kocht anschliessend 150 Minuten unter Rückfluss. Man kühlt ab, versetzt bei 0° bis 10° mit 21.0 ml 6.5N Natronlauge, filtriert und wäscht mehrmals mit je 20 ml Tetrahydrofuran nach. Abdestillieren des Lösungsmittels im Vakuum ergibt 10-Chlor-1,2,3,4,5,6-hexahydrobenzo[f]chinolin, das direkt weiterverarbeitet wird.

e) Man löst 20.1 g rohes 10-Chlor-1,2,3,4,5,6-hexahydrobenzo[f]chinolin in 40 ml Pyridin und 36 ml Essigsäureanhydrid, lässt das Reaktionsgemisch 20 Stunden bei Raumtemperatur stehen, dampft ein, nimmt den verbleibenden Rückstand zweimal in je 150 ml trockenem Toluol auf und dampft die erhaltenen Lösungen zur Trockene ein. Chromatographieren des Rückstandes an Kieselgel mit Chloroform liefert 4-Acetyl-10-chlor-1,2,3,4,5,6-hexahydrobezno[f]chinolin, das nach Um kristallisieren aus Isopropyläther einen Schmelzpunkt von 117-118° zeigt.

f) Zu einer Lösung von 8.50 g 4-Acetyl-10-chlor-1,2,3,4,5,6-hexahydrobenzo[f]chinolin in 205 ml Chloroform gibt man bei 0° bis 5° 16.0 g 85-prozentige m-Chlorperbenzoesäure in 205 ml Chloroform. Nach 4 Stunden Rühren bei Raumtemperatur versetzt man 4 g Kaliumjodid und 70 ml Wasser und bis zur Entfärbung mit Natriumthiosulfat. Die Chloroformphase wird mit 70 ml 2N Natronlauge und zweimal mit 170 ml Wasser gewaschen und im Vakuum eingedampft. Chromatographieren an 100 g Kieselgel mit Methylenchlorid liefert 4-Acetyl-9-chlor-1,2,4,5,6,7-hexahydro-4-benzazecin-3,8-dion mit einem Schmelzpunkt von 115-116° nach Umkristallisieren aus Isopropyläther.

g) Zu einer Lösung von 5.00 g (0.017 Mol) 4-Acetyl-9-chlor-1,2,4,5,6,7-hexahydrobenzazecin-3,8-dion in 153 ml Methanol gibt man 1 ml 25prozentige Salzsäure und rührt 4 Tage bei Raumtemperatur. Man engt die Lösung ein, extrahiert mit Methylenchlorid und wässriger Natriumhydrogencarbonat-Lösung, trocknet mit Natriumsulfat und destilliert das Lösungsmittel im Vakuum ab. Chromatographieren an Kieselgel mit Methylenchlorid und dann mit Essigester und Kristallisieren aus Petroläther ergibt 2-(4-Acetamidobutyryl)-3-chlor-hydrozimtsäuremethylester als weisse Kristalle mit Schmelzpunkt 51-52°.

Beispiel 12

a) Zu 230 g Aluminiumchlorid in 1050 ml Methylenchlorid tropft man unter Rühren zwischen 0° und 5° innerhalb von 60 Minuten 149.4 g 4-Fluorphenylessigsäurechlorid in 300 ml Methylenchlorid. Bei 0 bis 5° leitet man 30 Minuten lang Aethylen ein, rührt 1 Stunde bei Raumtemperatur weiter und versetzt bei 0° bis 5° innerhalb von 30 Minuten mit 600 ml Eiswasser. Die Methylenchloridphase wäscht man mit 2N Salzsäure, Wasser und gesättigter Natriumhydrogencarbo natlösung, trocknet mit Natriumsulfat und destilliert das Lösungsmittel im Vakuum ab. Man versetzt den Rückstand mit 250 ml tiefsiedenden Petroläther, lässt über Nacht im Eisschrank stehen und filtriert das 6-Fluor-3,4-dihydro-2(1H)-naphthalinon mit Schmelzpunkt 50-60° ab.

b) Man kocht 16.7 g 6-Fluor-3,4-dihydro-2(1H)-naphthalinon in 200 ml Benzol mit 8.4 ml Pyrrolidin und 0.35 g wasserfreier p-Toluolsulfonsäure 2,5 Stunden am Rückfluss. Das erhaltene 1-(6-Fluor-3,4-dihydro-2-naphthyl)pyrrolidin wird ohne weitere Reinigung mit 10,8 g Acrylamid und 0.5 g p-Toluolsulfonsäure versetzt. Man erhitzt unter Stickstoff je 2 Stunden bei 100° und 150°. Man löst in 180 ml Chloroform, wäscht mit Wasser und chromatographiert mit Chloroform über 150 g Kieselgel. Umkristallisieren aus Essigsäureäthylester ergibt 8-Fluor-1,4,5,6-tetrahydrobenzo[f]chinolin-3(2H)-on mit Schmelzpunkt 223-224°.

c) Zu einer gerührten Suspension von 2.17 g Lithiumaluminiumhydrid in 60 ml trockenem Tetrahydrofuran gibt man unter Stickstoff innerhalb von 35 Minuten bei 20° bis 25° portionenweise 6.20 g 8-Fluor-1,4,5,6,-tetrahydrobenzo[f]chinolin-3(2H)-on zu. Das Reaktionsgemisch wird 150 Minuten unter Rückfluss gekocht und dann bei 0° bis 10° mit 7,0 ml 6.5N Natronlauge versetzt. Man filtriert, wäscht mehrmals mit je 20 ml Tetrahydrofuran nach und destilliert das Lösungsmittel im Vakuum ab. Das so erhaltene 8-Fluor-1,2,3,4,5,6-hexahydrobenzo[f]chinolin wird direkt weiterverarbeitet.

d) Eine Lösung aus 6.00 g rohem 8-Fluor-1,2,3,4,5,6-hexahydrobenzo[f]chinolin in 13 ml Pyridin und 12 ml Essigsäureanhydrid lässt man 20 Stunden bei Raumtemperatur stehen, dampft ein, nimmt den verbleibenden Rückstand zweimal in je 50 ml trockenem Toluol auf und dampft die erhaltenen Lösungen zur Trockene ein. Der Rückstand wird mit Methylenchlorid an 150 g Kieselgel chromatographiert. Das erhaltene 4-Acetyl-8-fluor-1,2,3,4,5,6-hexahydrobenzo [f]chinolin zeigt nach Umkristallisieren aus Isopropyläther einen Schmelzpunkt von 101-102°.

e) Man versetzt 2.48 g 4-Acetyl-8-fluor-1,2,3,4,5,6-hexahydrobenzo[f]chinolin in 60 ml Chloroform bei 0° bis 5° mit 5.24 g 85-prozentiger m-Chlorperbenzoesäure in 40 ml Chloroform und rührt 3 Stunden bei Raumtemperatur. Dann gibt man 1.10 g Kaliumjodid und 15 ml Wasser zu und bis zur Entfärbung Natriumthiosulfat. Die Chloroformphase trennt man ab und wäscht mit 15 ml 2N Natronlauge und 2 x 40 ml Wasser. Abdestillieren des Chloroforms im Vakuum und Umkristallisieren aus Essigester liefert 4-Acetyl-11-fluor-1,2,4,5,6,7-hexahydro-4-benzazecin-3,8-dion mit einem Schmelzpunkt von 161-162°.

f) Zu einer Lösung von 3.00 g (0.011 Mol) 4-Acetyl-11-fluor-1,2,4,5,6,7-hexahydro-4-benzazecin-3,8-dion in 40 ml Acetonitril gibt man 20 ml 2N Salzsäure und rührt 65 Stunden bei Raumtemperatur. Einengen der Lösung, Chromatographieren an Kieselgel mit Tetrahydrofuran und Kristallisieren aus Aether ergibt 2-(4-Acetamidobutyryl)-5-fluorhydrozimtsäure als weisse Kristalle mit Schmelzpunkt 100-101°.

Beispiel 13

Zu einer Lösung von 5.00 g (0.018 Mol) 4-Acetyl-11-fluor-1,2,4,5,6,7-hexahydro-4-benzazecin-3,8-dion in 163 ml Methanol gibt man 0.75 ml 25prozentige Salzsäure und rührt 99 Stunden bei Raumtemperatur. Man engt die Lösung ein, extrahiert mit Toluol und gesättigter Natriumhydrogencarbonat-Lösung, trocknet mit Natriumsulfat und destilleirt das Lösungsmittel im Vakuum ab. Chromatographieren an Kieselgel mit Chloroform/Essigester und dann an Aluminiumoxid mit Methylenchlorid/Essigester und anschliessendes Verrühren in Petroläther ergibt 2-(4-Acetamidobutyryl)-5-fluorhydrozimtsäuremethylester als leicht gelbe Kristalle mit Schmelzpunkt 39-40°.

Beispiel 14

a) Man kocht 12.0 g 6-Methoxy-2-tetralon in 200 ml Benzol mit 5.6 ml Pyrrolidin in Gegenwart von 0.5 g Wasserfreier p-Toluolsulfonsäure 2 Stunden am Rückfluss. Das Toluol wird im Vakuum entfernt. Zum so erhaltenen rohen 1-(6-Methoxy-3,4-dihydro-2-naphtyl)pyrrolidin werden 9.70 g Acrylamid und 0.5 g p-Toluolsulfonsäure zugegeben. Man erhitzt unter Stickstoff je 2 Stunden bei 100° und 150°. extrahiert mit Chloroform/Wasser, trocknet mit Natriumsulfat und destilliert das Lösungsmittel im Vakuum ab. Chromatographieren an 160 g Kieselgel mit Methylenchlorid und Umkristallisieren aus Essigester ergibt 8-Methoxy-1,4,5,8-tetrahydrobenzo[f]chinolin-3(2H)-on mit Schmelzpunkt 208-209°.

b) Zu einer gerührten Suspension von 1.69 g Lithiumaluminiumhydrid in 50 ml trockenen Tetrahydrofuran werden bei 20° unter Stickstoff innerhalb von 35 Minuten portionenweise 5.10 g 8-Methoxy-1,4,5,8-tetrahydrobenzo[f]chinolin-3(2H)-on zugegeben. Das Reaktionsgemisch kocht 150 Minuten unter Rückfluss und wird bei 0° bis 10° mit 1.5 ml 6.5N Natronlauge versetzt. Man filtriert, wäscht mehrmals mit Tetrahydrofuran nach und destilliert das Lösungsmittel im Vakuum ab. Das so erhaltene 8-Methoxy-1,2,3,4,5,6-hexahydrobenzo[f]chinolin wird direkt weiterverarbeitet.

c) Man lässt eine Mischung aus 4.70 g 8-Methoxy-1,2,3,4,5,6-hexahydrobenzo[f]chinolin in 15 ml Pyridin und 11 ml Essigsäureanhydrid 20 Stunden bei Raumtemperatur stehen, dampft ein, nimmt den verbleibenden Rückstand zweimal in je 50 ml trockenem Toluol auf und dampft die erhaltenen Lösungen zur Trockene ein. Der Rückstand wird mit Methylenchlorid an 50 g Kieselgel chromatographiert und ergibt 4-Acetyl-8-methoxy-1,2,3,4,5,6-hexahydrobenzo[f]chinolin, mit einem Schmelzpunkt von 119-120° nach Umkristallisieren aus Isopropyläther.

d) Zu einer Lösung von 21.3 g 4-Acetyl-8-methoxy-1,2,3,4,5,6-hexahydrobenzo[f]chinolin in 200 ml Chloroform tropft man bei 0° bis 5° 38.0 g 85-prozentige m-Chlorperbenzoesäure in 250 ml Chloroform, und rührt 18 Stunden bei Raumtemperatur. Man versetzt mit Natriumjodid und Wasser und danach bis zur Entfärbung mit Natriumthiosulfat. Die Chloroformphase wird mit wässrigem Ammoniak und Natriumchloridlösung gewaschen, mit Natriumsulfat getrocknet und im Vakuum eingedampft. Man erhält 4-Acetyl-11-methoxy-1,2,4,5,6,7-hexahydro-4-benzazecin-3,8-dion mit einem Schmelzpunkt von 156-158° nach Umkristallisieren aus Essigester.

e) Zu einer Lösung von 5.50 g (0.019 Mol) 4-Acetyl-1,2,4,5,6,7-hexahydro-11-methoxy-4-benzazecin-3,8-dion in 150 ml Aethanol gibt man 5 ml konzentrierte Salzsäure und rührt über Nacht bei Raumtemperatur. Man engt die Lösung ein, extrahiert mit Methylenchlorid/Wasser, trocknet mit Magnesiumsulfat und destilliert das Lösungsmittel im Vakuum ab. Chromatographieren an Kieselgel mit Essigster und Kristallisieren aus Essigester/Aether ergibt 2-(Acetamidobutyryl)-5-methoxyhydrozimtsäureäthylester als weisse Kristalle mit Schmelzpunkt 59-61°.

Beispiel 15

a) Man löst 9.00 g (0.042 Mol) 6,7-Dichlor-3,4-dihydro-2(1H)naphthalinon und 0.3 g p-Toluolsulfonsäure in 200 ml Pyridin, tropft 3.5 ml (0.042 Mol) Pyrrolidin zu und kocht 2 Stunden unter Rückfluss. Abdestillieren des Lösungmittels im Vakuum, Zugabe von 200 ml Aether und Abfiltrieren der gebildeten Kristalle liefert 1-(6,7-Dichlor-3,4-dihydro-2-naphthyl)pyrrolidin mit Schmelzpunkt 141-142°.

b) Man rührt eine Schmelze aus 10.1 g (0.038 Mol) 1-(6,7-Dichlor-3,4-dihydro-1-naphthyl)pyrrolidin, 5.36 g (0.075 Mol) Acrylamid und 0.3 g p-Toluolsulfonsäure unter Stickstoff 2 Stunden bei 100° und 2 Stunden bei 150°. Umkristallisieren des Schmelzkuchens aus Aethanol liefert 8,9-Dichlor-1,4,5,6-tetrahydrobenzo[f]chinolin-3(2H)-on mit Schmelzpunkt 260-261°.

c) Man suspendiert unter Argon 1.57 g (0.042 Mol) Lithiumaluminiumhydrid in 60 ml Tetrahydrofuran, gibt langsam 5.56 g (0.021 Mol) 8,9-Dichlor-1,4,5,6-tetrahydrobenzo[f]chinolin-3(2H)-on zu und kocht 2,5 Stunden unter Rückfluss. Dann kühlt man auf 0° ab, tropft 5.2 ml 18prozentige Natronlauge zu und filtriert den gebildeten Niederschlag ab. Beim Eindampfen des Filtrats erhält man 8,9-Dichlor-1,2,3,4,5,6-hexahydrobenzo[f]chinolin als gelbes Oel, das direkt weiterverarbeitet wird.

d) Man löst 6.17 g (0.024 Mol) 8,9-Dichlor-1,2,3,4,5,6-hexahydrobenzo[f]chinolin in 10 ml Pyridin, tropft 9 ml (0.09 Mol) Essigsäureanhydrid zu und rührt 17 stunden bei Raumtemperatur. Chromatographieren an Kieselgel mit Chloroform liefert ein Produkt, das man in Aether suspendiert und abfiltriert. Man erhält 4-Acetyl-8,9-dichlor-1,2,3,4,5,6-hexahydrobezno[f]chinolin mit Schmelzpunkt 146-148°.

e) Man löst 4.14 g (0.014 Mol) 4-Acetyl-8,9-dichlor-1,2,3,4,5,6-hexahydrobenzo[f]chinolin in 70 ml Chloroform und tropft bei 0° eine Suspension von 7.28 g (0.036 Mol) m-Chlorperbenzoesäure (85-prozentig) in 100 ml Chloroform zu. Nach 3-stündigem Rühren bei Raumtemperatur filtriert man vom gebildeten Niederschlag ab und extrahiert mit Kaliumiodid- und Natriumthiosulfatlösung. Chromatographieren an Kieselgel mit Chloroform und Umkristallisieren aus Isopropyläther ergibt 4-Acetyl-10,11-dichlor-1,2,4,5,6,7-hexahydro-4-benzazecin-3,8-dion als weisse Kristalle mit Schmelzpunkt 163-165°.

f) Zu einer Lösung von 1.75 g (0.005 Mol) 4-Acetyl-10,11-dichlor-1,2,4,5,6,7-hexahydro-4-benzazecin-3,8-dion in 60 ml Aethanol gibt man 2 ml konzentrierte Salzsäure und rührt 20 Stunden bei Raumtemperatur. Man engt die Lösung ein, extrahiert mit Methylenchlorid/Wasser, trocknet mit Magnesiumsulfat und destilliert das Lösungsmittel im Vakuum ab. Chromatographieren an Kieselgel mit Methylenchlorid und Kristallisieren aus t-Butylmethyläther ergibt 2-(4-Acetamidobutyryl)-4,5-dichlor-hydrozimtsäureäthylester als weisse Kristalle mit Schmelzpunkt 63-65°.

## Beispiel 16

a) Man kocht eine Suspension aus 127 g (0.7 Mol) 6-Chlor-2-tetralon, 70.0 g (0.7 Mol) 3,3-Dimethylacrylamid, 63.6 ml (0.42 Mol) Tetramethoxysilan und 85.3 g (0.56 Mol) Caesiumfluorid in 400 ml Toluol während 18 Stunden unter Rückfluss. Abdestillieren des Lösungsmittels im Vakuum, Extrahieren mit Methylenchlorid/Wasser, Chromatographieren an Kieselgel mit Essigester/Hexan (1:1) und Umkristallisieren aus Methylenchlorid/Hexan liefert 8-Chlor-1,1-dimethyl-1,2,5,6-tetrahydrobenzo[f]chinolin-3-on als weisse Kristalle mit Schmelzpunkt 213°.

b) Man suspendiert unter Argon 3.36 g (0.089 Mol) Lithiumaluminiumhydrid in 100 ml Tetrahydrofuran, gibt langsam 11.6 g (0.044 Mol) 8-Chlor-1,1-dimethyl-1,2,5,6-tetrahydrobenzl[f]chinolin-3-on zu und kocht 2.5 Stunden unter Rückfluss. Dann kühlt man auf 0° ab, tropft 12 ml 18prozentige Natronlauge zu und filtriert. Beim Eindampfen des Filtrats erhält man 8-Chlor-1,1-dimethyl-1,2,3,4,5,6-hexahydrobenzo[f]-chinolin als gelbes Oel, welches direkt weiterverarbeitet wird.

c) Man löst 10.6 g (0.043 Mol) 8-Chlor-1,1-dimethyl-1,2,3,4,5,6-hexahydrobenzo[f]chinolin und 6.6 ml (0.047 Mol) Triäthylamin in 100 ml Methylenchlorid und tropft bei 0° 3.5 ml (0.047 Mol) Acetylbromid, gelöst in 20 ml Methylenchlorid, zu. Nach 1-stündigem Rühren extrahiert man mit Methylenchlo rid/Wasser, trocknet mit Magnesiumsulfat und destilliert das Lösungsmittel im Vakuum ab. Umkristallisieren aus Methylenchlorid/Hexan ergibt 4-Acetyl-8-chlor-1,1,-dimethyl-1,2,3,4,5,6-hexahydrobenzo[f]chinolin als weisse Kristalle mit Schmelzpunkt 126-127°.

d) Man löst 5.30 g (0.018 Mol) 4-Acetyl-8-chlor-1,1-dimethyl-1,2,3,4,5,6-hexahydrobenzo[f]chinolin in 100 ml Methylenchlorid und tropft bei 0° eine Suspension von 8.20 g (0.04 Mol) m-Chlorperbenzoesäure (85-prozentig) in 50 ml Methylenchlorid zu. Nach 1.5 Stunden Rühren bei Raumtemperatur filtriert man vom gebildeten Niederschlag ab und extrahiert mit Methylenchlorid/Wasser. Chromatographieren an Kieselgel mit Essigester/Hexan (1:1) und Umkristallisieren aus Aether/Hexan ergibt 4-Acetyl-11-chlor-7,7-dimethyl-1,2,4,5,6,7-hexahydro-4-benzazecin-3,8-dion als weisse Kristalle mit Schmelzpunkt 113-115°.

e) Zu einer Lösung von 2.40 g (0.008 Mol) 4-Acetyl-11-chlor-7,7-dimethyl-1,2,4,5,6,7-hexahydro-4-benzazecin-3,8-dion in 150 ml Methanol gibt man 3 ml konzentrierte Salzsäure und rührt 24 Stunden bei Raumtemperatur. Man engt die Lösung ein, extrahiert mit Methylenchlorid/Wasser, trocknet mit Magnesiumsulfat und destilliert das Lösungsmittel im Vakuum ab. Chromatographieren an Kieselgel mit Hexan-Essigester (2:1) und dann an Aluminiumoxid mit Essigester ergibt 2-(4-Acetamido-2,2-dimethylbutyryl)-5-chlor-hydrozimtsäuremethylester als gelbes Oel.

## Beispiel 17

In einem Stahlautoklaven gibt man zu einer Lösung von 10.00 g (0.032 Mol) 2-(4-Acetamidobutyryl)-5-chlorhydrozimtsäure in 40 ml Tetrahydrofuran 0.3 ml konzentrierte Schwefelsäure und kondensiert ca. 60 ml Isobutylen ein. Man rührt 30 Tage bei Raumtemperatur, engt die Lösung ein, extrahiert mit Methylenchlorid/Wasser, trocknet mit Magnesiumsulfat und destilliert das Lösungsmittel im Vakuum ab. Chromatographieren mit Essigester an Aluminiumoxid und dann an Kieselgel ergibt 2-(4-Acetamidobutyryl)-5-chlorhydrozimtsäure-t-butylester als farbloses Oel.

Beispiel 18

Man rührt 2.00 g (0.006 Mol) 2-(4-Acetamidobutyryl)-5-chlorhydrozimtsäure und 0.5 ml (0.006 Mol) Thionylchlorid in 20 ml Methylenchlorid 5 Minuten bei Raumtemperatur und gibt dann bei 0° eine Lösung von 2.2 ml (0.023 Mol) 3-Hydroxymethylpyridin in 20 ml Methylenchlorid zu. Nach 1 Stunde Rühren bei Raumtemperatur engt man die Lösung ein, extrahiert mit Methylenchlorid/Wasser, trocknet mit Magnesiumsulfat und destilliert das Lösungsmittel im Vakuum ab. Chromatographieren an Aluminiumoxid mit Essigester ergibt 2-(4-Acetamidobutyryl)-5-chlorhydrozimtsäure-3-pyridylmethylester als beiges Oel.

Beispiel 19

Man rührt 8.15 g (0.025 Mol) 2-(4-Acetamidobutyryl)-5-chlorhydrozimtsäuremethylester in 200 ml einer Lösung von Ammoniak in Methanol 30 Stunden bei Raumtemperatur. Nach Einengen der Lösung extrahiert man mit Methylenchlorid/Wasser, trocknet mit Magnesiumsulfat, destilliert das Lösungsmittel im Vakuum ab und chromatographiert an Kieselgel mit Chloroform-Methanol (9:1). Umkristallisieren aus Methanol/Aether liefert 2-(4-Acetamidobutyryl)-5-chlor-hydrozimtsäureamid als weisse Kristalle mit Schmelzpunkt 149-151°.

Beispiel 20

Man rührt 1.00 g (0.002 Mol) 2-[4-(4-Acetamidobutyramido)butyryl]-5-chlorhydrozimtsäureäthylester in 20 ml einer Lösung von Ammoniak in Methanol 6 Tage bei Raumtemperatur. Nach Einengen der Lösung extrahiert man mit Methylenchlorid/ Wasser, trocknet mit Magnesiumsulfat, destilliert das Lösungsmittel im Vakuum ab und chromatographiert an Aluminiumoxid mit Essigester. Umkristallisieren aus Methanol/t-Butylmethyläther liefert 2-[4-(4-Acetamidobutyramido)butyryl]-5-chlor-hydrozimtsäureamid als weisse Kristalle mit Schmelzpunkt 126-127°.

Beispiel 21

Man rührt 2.50 g (0.008 Mol) 2-(4-Acetamidobutyryl)-3-chlor-hydrozimtsäuremethylester in 100 ml einer Lösung von Ammoniak in Methanol 2 Tage bei Raumtemperatur. Nach Einengen der Lösung extrahiert man mit Methylenchlorid/Wasser, trocknet mit Magnesiumsulfat und destilliert das Lösungsmittel im Vakuum ab. Umkristallisieren aus Methanol/Aether liefert 2-(4-Acetamidobutyryl)-3-chlor-hydrozimtsäureamid als weisse Kristalle mit Schmelzpunkt 148-150°.

Beispiel 22

Man rührt 9.30 g (0.03 Mol) 2-(4-Acetamidobutyryl)-5-fluor-hydrozimtsäuremethylester in 200 ml einer Lösung von Ammoniak in Methanol 6 Tage bei Raumtemperatur. Nach Einengen der Lösung extrahiert man mit Methylenchlorid/Wasser, trocknet mit Magnesiumsulfat, destilliert das Lösungsmittel im Vakuum ab und chromatographiert an Kieselgel mit Methylenchlorid-Methanol (20:1). Umkristallisieren aus Methanol/Aether liefert 2-(4-Acetamidobutyryl)-5-fluor-hydrozimtsäureamid als weisse Kristalle mit Schmelzpunkt 125-127°.

Beispiel 23

Man rührt 3.10 g (0.01 Mol) 2-(4-Acetamidobutyryl)-5-chlorhydrozimtsäure und 1.70 g (0.015 Mol) 1,1′-Carbonyldiimidazol in 30 ml Tetrahydrofuran 1 Stunde bei Raumtemperatur, gibt bei -70° 1.1 ml (0.015

Mol) Diäthylamin zu und lässt über Nacht auf Raumtemperatur aufwärmen. Nach Einengen der Lösung extrahiert man Methylenchlorid/Wasser, trocknet mit Magnesiumsulfat und destilliert das Lösungsmittel im Vakuum ab. Chromatographieren an Kieselgel mit Essigester ergibt 2-(4-Acetamidobutyryl)-5-chlor-N,N-diäthylhydrozimtsäureamid als gelbes Oel.

## Beispiel 24

Man rührt 3.10 g (0.01 Mol) 2-(4-Acetamidobutyryl)-5-chlorhydrozimtsäure und 1.70 g (0.011 Mol) 1,1'-Carbonyldiimidazol in 30 ml Tetrahydrofuran 1 Stunde bei Raumtemperatur, gibt bei -70° 1.2 ml (0.011 Mol) 1-Methylpiperazin zu und lässt über Nacht auf Raumtemperatur aufwärmen. Nach Einengen der Lösung extrahiert man zuerst mit Methylenchlorid/2N Salzsäure und dann mit Methylenchlorid konzentrierter wässriger Ammoniaklösung, trocknet mit Natriumsulfat und destilliert das Lösungsmittel im Vakuum ab. Chromatographieren an Kieselgel mit Methylenchlorid-Methanol (20:1) und Kristallisieren aus Essigester/Hexan ergibt N-[3-[4-Chlor-2-[2-[(4-methyl-piperazin-1-yl)carbonyl]äthyl]benzoyl]propyl]acetamid als weisse Kristalle mit Schmelzpunkt 126-128°.

## Beispiel 25

Man rührt 3.75 g (0.012 Mol) 2-(4-Acetamidobutyryl)-5-chlorhydrozimtsäure und 2.05 g (0.013 Mol) 1,1'-Carbonyldiimidazol in 40 ml Tetrahydrofuran 1 Stunde bei Rückfluss und gibt dann 1.67 ml (0.013 Mol) 2-Piperidinoäthylamin zu. Nach 2-stündigem Rühren bei Rückflusstemperatur engt man die Lösung ein, extrahiert mit Essigester/Wasser (pH14), trocknet mit Natriumsulfat und destilliert das Lösungsmittel im Vakuum ab. Chromatographieren an Kieselgel mit Chloroform-Methanol (4:2) und dann an Aluminiumoxid mit Essigester-Methanol (98:2) und anschliessendes Kristallisieren aus Essigester/Cyclohexan ergibt 2-(4-Acetamidobutyryl)-5-chlor-N-(2-piperidinoäthyl)hydrozimtsäureamid als weisse Kristalle mit Schmelzpunkt 103-105°.

## Beispiel 26

Man rührt 3.10 g (0.01 Mol) 2-(4-Acetamidobutyryl)-5-chlorhydrozimtsäure und 1.70 g (0.015 Mol) 1,1'-Carbonyldiimidazol in 30 ml Tetrahydrofuran 1 Stunde bei Raumtemperatur und gibt dann 1.35 g (0.015 Mol) 2-Phenyläthylamin zu. Nach 2-stündigem Rühren bei Raumtemperatur engt man die Lösung ein, extrahiert mit Methylenchlorid/Wasser, trocknet mit Magnesiumsulfat und destilliert das Lösungsmittel im Vakuum ab. Kristallisieren aus Essigester/Hexan ergibt 2-(4-Acetamidobutyryl)-5-chlor-N-phenäthyl-hydrozimtsäureamid als weisse Kristalle mit Schmelzpunkt 127-128°.

## Beispiel 27

Man rührt 5.00 g (0.016 Mol) 2-(4-Acetamidobutyryl)-5-chlorhydrozimtsäure, 1.26 g (0.013 Mol) 4-Aminopyridin und 4.16 g N,N'-Dicyclohexylcarbodiimid in 100 ml Tetrahydrofuran 18 Stunden bei Raumtemperatur, engt die Lösung ein, extrahiert mit Essigester/Wasser, trocknet mit Magnesiumsulfat und destilliert das Lösungsmittel im Vakuum ab. Chromatographieren an Kieselgel mit Essigester-Methanol (4:1) und Kristallisieren aus Methanol/t-Butylmethyläther ergibt 2-[4-Acetamidobutyryl]-5-chlor-N-(4-pyridyl)-hydrozimtsäureamid als weisse Kristalle mit Schmelzpunkt 170-171°.

Beispiel 28

Man rührt 2.00 g (0.006 Mol) 2-(4-Acetamidobutyryl)-5-chlorhydrozimtsäure und 1.09 g (0.007 Mol) 1,1'-Carbonyldiimidazol in 20 ml Tetrahydrofuran 1,5 Stunden bei Raumtemperatur und gibt dann 1.04 g (0.007 Mol) L-Alaninäthylesterhydrochlorid und 0.94 ml (0.007 Mol) Triäthylamin zu. Nach 2-stündigem Rühren bei Raumtemperatur extrahiert man mit Methylenchlorid/Wasser, trocknet mit Magnesiumsulfat und destilliert das Lösungsmittel im Vakuum ab. Chromatographie ren an Kieselgel mit Essigester-Methanol (9:1) und dann an Aluminiumoxid mit Essigester und anschliessendes Kristallisieren aus Essigester Hexan ergibt N-[2-(4-Acetamidobutyryl)-5-chlorhydrocinnamoyl]-L-alaninäthylester als weisse Kristalle mit Schmelzpunkt 106°.

Beispiel 29

Man rührt 3.10 g (0.01 Mol) 2-(4-Acetamidobutyryl)-5-chlorhydrozimtsäure und 1.70 g (0.015 Mol) 1,1'-Carbonyldiimidazol in 50 ml Tetrahydrofuran 1 Stunde bei Raumtemperatur und gibt dann 2.51 g (0.011 Mol) L-Glutaminsäurediäthylesterhydrochlorid und 1.8 ml (0.011 Mol) N-Aethyldiisopropylamin zu. Nach 2 Stunden Rühren bei Raumtemperatur engt man die Lösung ein, extrahiert mit Methylenchlorid/Wasser, trocknet mit Natriumsulfat und destilliert das Lösungsmittel im Vakuum ab. Chromatographieren an Aluminiumoxid mit Methylenchlorid-Methanol (97:3) und Kristallisieren aus Essigester/Hexan ergibt N-[2-(4-Acetamidobutyryl)-5-chlorhydrocinnamoyl]-L-glutaminsäurediäthylester als weisse Kristalle mit Schmelzpunkt 109-111°.

Beispiel 30

Man rührt 1.75 g (0.006 Mol) 2-(4-Acetamidobutyryl)-5-chlorhydrozimitsäure und 0.95 g (0.006 Mol) 1,1'-Carbonyldiimidazol in 15 ml Tetrahydrofuran bei Raumtemperatur und gibt dann 1.0 ml (0.006 Mol) N-Aethyldiisopropylamin und 0.82 g (0.006 Mol) Glycinäthylester-hydrochlorid zu. Nach 1-stündigem Rühren bei Raumtemperatur engt man die Lösung ein, extrahiert mit Methylenchlorid/Wasser, trocknet mit Magnesiumsulfat und destilliert das Lösungsmittel im Vakuum ab. Chromatographieren an Aluminiumoxid mit Essigester-Aethanol (9:1) und Kristallisieren aus Methylenchlorid/t-Butylmethyläther ergibt N-[2-(4-Acetamidobutyryl)-5-chlorhydrocinnamoyl]glycinäthylester als weisse Kristalle mit Schmelzpunkt 96°.

Beispiel 31

Man rührt 3.00 g (0.008 Mol) N-[2-(4-Acetamidobutyryl)-5-chlorhydrocinnamoyl]glycinäthylester in 30 ml einer Lösung von Ammoniak in Methanol 3 Tage bei Raumtemperatur. Nach Einengen der Lösung extrahiert man mit Methylenchlorid/Wasser, trocknet mit Magnesiumsulfat und destilliert das Lösungsmittel im Vakuum ab. Umkristallisieren aus Methanol/t-Butylmethyläther liefert 2-(4-Acetamidobutyryl)-N-(carbamolymethyl)-5-chlorhydrozimtsäureamid als weisse Kristalle mit Schmelzpunkt 148-149°.

Beispiel 32

Zu einer Lösung von 3.25 g (0.01 Mol) 2-(4-Acetamidobutyryl)-5-chlor-hydrozimtsäuremethylester in 35 ml Methanol gibt man 0.38 g (0.01 Mol) Natriumborhydrid und rührt 1 Stunde bei Raumtemperatur. Man engt die Lösung ein, extrahiert mit Methylenchlorid/Wasser, trocknet mit Magnesiumsulfat und destilliert das Lösungsmittel im Vakuum ab. Chromatographieren an Kieselgel mit Chloroform-Methanol (20:1) ergibt 2-(4-Acetamido-1-hydroxybutyl)-5-chlorhydrozimtsäuremethylester als gelbes Oel.

## Beispiel 33

Man rührt eine Suspension von 15.5 g (0.05 Mol) 2-(4-Acetamidobutyryl)-5-chlor-hydrozimtsäure und 8.50 g (0.052 Mol) 1,1′-Carbonyldiimidazol in 350 ml Tetrahydrofuran 2 Stunden bei Raumtemperatur und gibt 4.00 g (0.052 Mol) Glykolsäureamid zu. Nach Rühren über Nacht bei Raumtemperatur engt man die Lösung ein, extrahiert mit Methylenchlorid/Wasser, trocknet mit Magnesiumsulfat und destilliert das Lösungmittel im Vakuum ab. Chromatographieren an Kieselgel mit Methylenchlorid-Methanol (20:1) und Kristallisieren aus Essigester/Aether ergibt 2-(4-Acetamidobutyryl)-5-chlorhy drozimtsäurecarbamoylmethylester als weisse Kristalle mit Schmelzpunkt 114-116°.

## Beispiel 34

Man suspendiert 3.10 g (0.01 Mol) 2-(4-Acetamidobutyryl)-5-chlorhydrozimtsäure und 1.70 g (0.01 Mol) 1,1′-Carbonyldiimidazol in 30 ml Tetrahydrofuran, rührt 1 Stunde bei Raumtemperatur und versetzt mit 2.50 g (0.01 Mol) N-(2-Aminoäthyl)-5-chlor-2-pyridincarboxamid-hydrochlorid und 1.8 ml (0.01 Mol) Aethyldiisopropylamin. Nach 2 Stunden wird eingeengt, mit Essigester/Wasser extrahiert und aus Methanol/Aether umkristallisiert. Das erhaltene 2-(4-Acetamidobutyryl)-5-chlor-N-[2-(5-chlor-2-pyridincarboxamido)äthyl]-hydrozimtsäureamid schmilzt bei 163-165°.

## Beispiel 35

a) Man suspendiert 8.00 g (0.06 Mol) Aluminiumchlorid in 600 ml Methylenchlorid, versetzt bei 0° mit 7.56 g (0.04 Mol) 4-Chlorphenylessigsäurechlorid in 20 ml Methylenchlorid und tropft bei -40° bis -50° eine Lösung von 4.17 g (0.04 Mol) Styrol in 400 ml Methylenchlorid zu. Nach Aufwärmen auf Raumtemperatur giesst man auf Eis, extrahiert mit Methylenchlorid, trocknet mit Magnesiumsulfat, filtriert über Dicalit und destilliert das Lösungsmittel im Vakuum ab. Chromatographieren an Kieselgel mit Essigester-Hexan (1:4) und Umkristallisieren aus Aether/Hexan ergibt 6-Chlor-3,4-dihydro-4-phenyl-2(1H)-naphthalinon als beige Kristalle mit Schmelzpunkt 61-62°.

b) Man versetzt eine Lösung aus 73.3 g (0.23 Mol) 6-Chlor-3,4-dihydro-4-phenyl-2(1H)-naphthalinon in 1,5 1 Toluol mit 32.4 g (0.58 Mol) Kaliumhydroxid und 83.7 g (0.64 Mol) 3-Chlorpropylamin-hydrochlorid und kocht 42 Stunden am Wasserabscheider. Nach Abkühlen auf Raumtemperatur gibt man 79 ml (0.56 Mol) Triäthylamin und tropfenweise 29.7 ml (0.42 Mol) Acetylchlorid in 250 ml Toluol zu, rührt 1 Stunde, extrahiert mit Essigester/Wasser, trocknet mit Magnesiumsulfat und destilliert das Lösungsmittel im Vakuum ab. Chromatographieren über Kieselgel mit Hexan-Essigester (4:1) und dann mit Hexan-Essigester (1:1) und Umkristallisieren aus Essigester ergibt 4-Acetyl-8-chlor-1,2,3,4,5,6-hexahydro-6-phenylbenzo[f]chinolin als beige Kristalle mit Schmelzpunkt 167-168°.

c) Zu einer Lösung von 6.60 g (0.02 Mol) 4-Acetyl-8-chlor-1,2,3,4,5,6-hexahydro-6-phenylbenzo[f]-chinolin in 70 ml Methylenchlorid gibt man 8.13 g (0.04 Mol) m-Chlorperbenzoesäure (85-prozentig) in 80 ml Methylenchlorid und rührt 1 Stunde bei Raumtemperatur. Man filtriert, extrahiert mit Methylenchlorid/gesättigter Natriumhydrogencarbonat-Lösung, trocknet mit Magnesiumsulfat und destilliert das Lösungsmittel im Vakuum ab. Kristallisieren aus Methylenchlorid/Hexan ergibt 4-Acetyl-11-chlor-1-phenyl-1,2,4,5,6,7-hexahydro-4-benzazecin-3,8-dion als weisse Kristalle mit Schmelzpunkt 184-185°

d) Man rührt eine Lösung von 3.30 g (0.009 Mol) 4-Acetyl-11-chlor-1-phenyl-1,2,4,5,6,7-hexahydro-4-benzazecin-3,8-dion in 40 ml Tetrahydrofuran und 20 ml 2N Salzsäure zwei Tage bei Raumtemperatur, engt die Lösung ein, extrahiert mit Methylenchlorid/Wasser, trocknet mit Magnesiumsulfat und destilliert das Lösungsmittel im Vakuum ab. Chromatographieren an Kieselgel mit Hexan-Essigester (1:1) und Kristallisieren aus Essigester/Hexan ergibt 2-(4-Acetamidobutyryl)-5-chlor-β-phenylhydrozimtsäure als weisse Kristalle mit Schmelzpunkt 114-116°.

Beispiel 36

Zu einer Suspension von 2.00 g (0.005 Mol) 2-(4-Acetamidobutyryl)-5-chlor-β-phenylhydrozimtsäure in 120 ml Wasser gibt man 5.2 ml (0.005 Mol) 1N Natronlauge, versetzt langsam mit 0.88 g (0.005 Mol) Silbernitrat in 1 ml Wasser und rührt über Nacht bei Raumtemperatur. Der gebildete Niederschlag wird abfiltriert und mit 50 ml Aethyliodid über Nacht gerührt. Man extrahiert mit Methylenchlorid/Wasser, trocknet mit Magnesiumsulfat und destilliert das Lösungsmittel im Vakuum ab. Chromatographieren an Kieselgel mit Hexan-Essigester (1:1) und an Aluminiumoxid mit Hexan-Essigester (1:1) ergibt 2-(4-Acetamidobutyryl)-5-chlor-β-phenylhydrozimtsäureäthylester als farbloses Oel.

Beispiel 37

Zu einer Lösung von 23.8 g (0.06 Mol) N-[3-[(11-Chlor-2,3,5,6,7,8-hexahydro-3,8-dioxo-4-benzazecin-4-(1H)-yl)-carbonyl]propyl]acetamid in 240 ml Tetrahydrofuran gibt man 120 ml 2N Salzsäure und rührt 8 Tage bei Raumtemperatur. Man engt die Lösung ein, extrahiert mit Methylenchlorid/Wasser, trocknet mit Magnesiumsulfat und destilliert das Lösungsmittel im Vakuum ab. Umkristallisieren aus Methanol/Aether ergibt 2-[4-(4-Acetamidobutyramido)butyryl]-5-chlorhydrozimtsäure als beige Kristalle mit Schmelzpunkt 108°

Beipsiel 38

Man rührt 6.00 g (0.015 Mol) 2-[4-(4-Acetamidobutyramido)butyryl]-5-chlorhydrozimtsäure und 2.57g (0.016 Mol) 1,1′-Carbonyldiimidazol in 60 ml Tetrahydrofuran 1,5 Stunden bei Raumtemperatur und gibt 2.22 g (0.016 Mol) Glycinäthylester-hydrochlorid und 2.2 ml (0.016 Mol) Triäthylamin zu. Nach 1 Stunde Rühren bei Raumtemperatur engt man die Lösung ein, extrahiert mit Methylenchlorid/Wasser, trocknet mit Magnesiumsulfat und destilliert das Lösungsmittel im Vakuum ab. Chromatographieren an Kieselgel mit Essigester-Aethanol (9:1) und Kristallisieren aus Methanol-Aether ergibt N-[2-[4-(4-Acetamidobutyramido)-butyryl]-5-chlorhydrocinnamoyl]-glycinäthylester als weisse Kristalle mit Schmelzpunkt 94-96°.

Beispiel 39

Man rührt 2.50 g (0.005 Mol) N-[2-[4-(4-Acetamidobutyramido)butyryl]-5-chlorhydrocinnamoyl]-glycinäthylester in 50 ml einer Lösung von Ammoniak in Methanol über Nacht bei Raumtemperatur. Nach Einengen der Lösung extrahiert man mit Methylenchlorid/Wasser, trocknet mit Magnesiumsulfat und destilliert das Lösungsmittel im Vakuum ab. Zweimaliges Umkristallisieren aus Methanol/Aether ergibt 2-[4-(4-Acetamidobutyramido)butyryl]-N-(carbamoylmethyl)-5-chlorhydrozimtsäureamid als weisse Kristalle mit Schmelzpunkt 172-174°.

Beispiel 40

Zu einer Lösung von 1.50 g (0.004 Mol) N-[2-(4-Acetamidobutyryl)-5-chlorhydrocinnamoyl]-glycinäthylester in 15 ml Tetrahydrofuran gibt man 15 ml 2N Natronlauge und rührt 1 Stunde bei Raumtemperatur. Nach Ansäuern mit 1.7 ml 2N Salzsäure extrahiert man mit Essigester/Wasser, trocknet mit Magnesiumsulfat und destillliert das Lösungsmittel im Vakuum ab. Umkristallisieren am Essigester/Aether liefert N-[-2-(4-Acetamidobutyryl)-5-chlorhydrocinnamoyl]glycin als weisse Kristalle mit Schmelzpunkt 88-89°.

Beispiel 41

a) Man löst 25.0 g (0.115 Mol) 8-Chlor-1,2,3,4,5,6-hexhydrobenzo[f]chinolin in 145 ml Methylenchlorid, tropft bei 0° 21.8 g (0.182 Mol) α-Chloracetylisocyanat in 30 ml Methylenchlorid zu und rührt über Nacht bei Raumtemperatur. Nach Zugabe von 200 ml Methanol rührt man erneut 24 Stunden, filtriert das gebildete 8-Chlor-N-(chloracetyl)-2,3,5,6-tetrahydrobenzo[f]chinolin-4(1H)-carboxamid ab, das nach Umkristallisieren aus Essigester/Aether einen Schmelzpunkt von 158-161° zeigt. Aus dem Filtrat und der Mutterlauge kann eine weitere Menge des obigen Produkts erhalten werden.

b) Man rührt eine Suspension aus 11.85 g (0.035 Mol) 8-Chlor-N-(chloracetyl)-2,3,5,6-tetrahydrobenzo[f]chinolin -4(1H)-carboxamid und 4.4 ml (0.091 Mol) Hydrazinhydrat in 250 ml Aethanol 3 Stunden bei Raumtemperatur, engt im Vakuum ein und extrahiert mit Methylenchlorid/Wasser. Trocknen mit Magnesiumsulfat und Abdestillieren des Lösungsmittels im Vakuum liefert 8-Chlor-2,3,5,6-tetrahydrobenzo-[f]chinolin-4(1H)-carboxamid als weisse Kristalle, die nach Umkristallisieren aus Methylenchlorid einen Schmelzpunkt von 169-171° zeigen.

c) Man suspendiert 9.20 g (0.035 Mol) 8-Chlor-2,3,5,6-tetrahydrobenzo[f]chinolin-4(1H)-carboxamid in 100 ml Methylenchlorid und tropft bei 10-15° 14.1 g (0.071 Mol) m-Chlorperbenzoesäure (85-prozentig) in 150 ml Methylenchlorid zu. Nach 1,5 Stunden Rühren bei Raumtemperatur giesst man auf gesättigte Natriumhydrogencarbonatlösung, extrahiert mit Methylenchlorid, trocknet mit Magnesiumsulfat und destilliert das Lösungsmittel im Vakuum ab. Der erhaltene Feststoff wird mit heissem Essigester gewaschen. Umkristallisieren aus Dioxan/Aether liefert 11-Chlor-2,3,5,6,7,8-hexahydro-3,8-dioxo-4-benzazecin-4(1H)-carboxamid als weisse Kristalle mit Schmelzpunkt 182-184°.

d) Zu einer Lösung von 6.80 g (0.023 Mol) 11-Chlor-2,3,5,6,7,8-hexahydro-3,8-dioxo-4-benzazecin-4-(1H)-carboxamid in 5 l Aethanol gibt man 7 ml konzentrierte Salzsäure und rührt 13 Tage bei Raumtemperatur. Man engt die Lösung ein, extrahiert mit Methylenchlorid/Wasser, trocknet mit Magnesiumsulfat und destilliert das Lösungsmittel im Vakuum ab. Chromatographieren an Kieselgel mit Methylenchlorid/Methanol (97:3) und Kristallisieren aus Essigester/Hexan ergibt 5-Chlor-2-(4-ureidobutyryl)hydrozimtsäureäthylester als weisse Kristalle mit Schmelzpunkt 127-129°.

Beispiel A

Man stellt Tabletten folgender Zusammensetzung her, welche als Wirkstoff N-[2-(4-Acetamidobutyryl)-5-chlorhydrocinnamoyl]glycinäthylester enthalten.

| | pro Tablette |
|---|---|
| 1. Wirkstoff (mikronisiert) | 50 mg |
| 2. Milchzucker | 120 mg |
| 3. Maisstärke | 50 mg |
| 4. Polyvinylpyrrolidon | 8 mg |
| 5. Natrium-carboxymethylstärke | 20 mg |
| 6. Magnesiumstearat | 2 mg |
| | 250 mg |

Der Wirkstoff wird mit einer Mischung aus Milchzucker und Maisstärke homogen vermischt. Man siebt, befeuchtet mit einer wässerigen Polyvinylpyrrolidonlösung, granuliert und trocknet. Das getrocknete Granulat wird mit Natrium-carboxymethylstärke und Magnesiumstearat vermischt, und die so erhaltene Mischung wird zu Tabletten geeigneter Grösse mit Bruchrille verpresst.

Beispiel B

Man stellt Tabletten folgender Zusammensetzung her, welche als Wirkstoff N-[2-(4-Acetamidobutyryl)-5-chlorhydrocinnamoyl]glycinäthylester enthalten.

| | pro Tablette |
|---|---|
| 1. Wirkstoff (mikronisiert) | 10 mg |
| 2. Milchzucker | 88 mg |
| 3. Mikrokristalline Cellulose | 60 mg |
| 4. Maisstärke | 20 mg |
| 5. Natrium-carboxymethylstärke | 20 mg |
| 6. Magnesiumstearat | 2 mg |
| | 200 mg |

Der Wirkstoff wird mit Milchzucker homogen vermischt. Man siebt, mischt dann eine Mischung aus mikrokristalliner Cellulose, Maisstärke und Natrium-carboxymethylstärke zu und vermengt mit dem Magnesiumstearat. Die so erhaltene pressfertige Mischung wird zu Tabletten geeigneter Grösse mit Bruchrille verarbeitet.

## Beispiel C

Man stellt Tabletten folgender Zusammensetzung her, welche als Wirkstoff N-[2-(4-Acetamidobutyryl)-5-chlorhydrocinnamoyl]glycinäthylester enthalten.

| | pro Tablette |
|---|---|
| 1. Wirkstoff (mikronisiert) | 0.10 mg |
| 2. Milchzucker | 126.90 mg |
| 3. Maisstärke | 50.00 mg |
| 4. Polyvinylpyrrolidon | 6.00 mg |
| 5. Natrium-carboxymethylstärke | 15.00 mg |
| 6. Magnesiumstearat | 2.00 mg |
| | 200.00 mg |

Der Wirkstoff wird mit einer Mischung aus Milchzucker und Maisstärke homogen vermischt. Man siebt, befeuchtet mit einer wässerigen Polyvinylpyrrolidonlösung, granuliert und trocknet. Das getrocknete Granulat wird mit Natrium-carboxymethylstärke und Magnesiumstearat vermischt, und die so erhaltene Mischung wird zu Tabletten geeigneter Grösse mit Bruchrille verpresst.

## Ansprüche

1. Hydrozimtsäurederivate der allgemeinen Formel

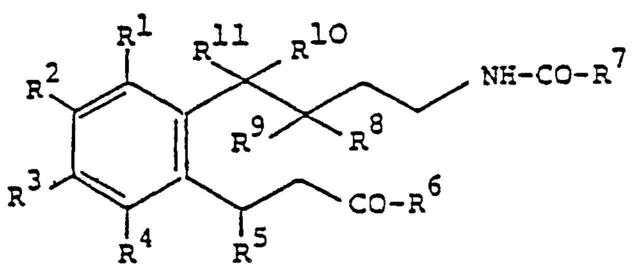

I

worin

- von den Symbolen $R^1$ bis $R^4$ eines oder zwei Halogen oder Methoxy und die übrigen Wasserstoff;
- $R^5$ Wasserstoff oder Phenyl;
- $R^6$ einen Rest der Formel $-OR^{12}$ (a) oder $-NR^{13}R^{14}$ (b);

- $R^7$ $(C_1-C_4)$-Alkyl, $(C_2-C_5)$-Alkanoylamino-$(C_2-C_5)$-alkyl, Amino oder $(C_1-C_4)$-Alkoxyphenyl;
- $R^8$ und $R^9$ je Wasserstoff oder $(C_1-C_4)$-Alkyl;
- $R^{10}$ Wasserstoff und $R^{11}$ Hydroxy oder $R^{10}$ und $R^{11}$ zusammen Oxo;
- $R^{12}$ Wasserstoff, $(C_1-C_{10})$-Alkyl, Pyridylmethyl oder Carbamoylmethyl;
- $R^{13}$ Wasserstoff, $(C_1-C_4)$-Alkyl und $R^{14}$ Wasserstoff, $(C_1-C_4)$-Alkyl, Pyridyl, Phenyl-$(C_1-C_4)$-alkyl, Carboxy-$(C_1-C_4)$-alkyl, Carbamoyl-$(C_1-C_4)$-alkyl, $(C_1-C_4)$-Alkoxycarbonyl-$(C_1-C_4)$-alkyl, Di-$(C_1-C_4)$-alkoxycarbonyl-$(C_2-C_5)$-alkyl, Piperidino-$(C_2-C_4)$-alkyl oder Halopyridincarboxamido-$(C_2-C_4)$-alkyl oder $R^{13}$ und $R^{14}$ zusammen mit dem Stickstoffatom 4-$(C_1-C_4)$-Alkyl-piperazin-1-yl bedeuten,

sowie pharmazeutisch anwendbare Salze von basischen Verbindungen der Formel I mit Säuren bzw. von sauren Verbindungen der Formel I mit Basen.

2. Verbindungen gemäss Anspruch 1, worin $R^4$ Wasserstoff bedeutet und entweder $R^1$ Chlor und $R^2$ und $R^3$ Wasserstoff oder $R^1$ Wasserstoff und $R^2$ und $R^3$ Chlor oder $R^1$ und $R^2$ Wasserstoff und $R^3$ Fluor, Chlor oder Methoxy bedeuten.

3. Verbindungen gemäss Anspruch 2, worin $R^3$ Chlor und $R^1$, $R^2$ und $R^4$ Wasserstoff bedeuten.

4. Verbindungen gemäss einem der Ansprüche 1 bis 3, worin $R^5$ Wasserstoff bedeutet.

5. Verbindungen gemäss einem der Ansprüche 1 bis 4, worin $R^6$ einen Rest der in Anspruch 1 definierten Formel (a) oder (b) bedeutet, wobei $R^{12}$ Wasserstoff, Methyl, Aethyl, n-Propyl, Isopropyl, n-Butyl, tert.-Butyl, n-Nonyl, 5-Nonyl, 3-Pyridylmethyl oder Carbamoylmethyl bedeutet bzw. entweder $R^{13}$ und $R^{14}$ beide Wasserstoff oder beide Aethyl oder zusammen mit dem Stickstoffatom 4-Methylpiperazin-1-yl bedeuten oder $R^{13}$ Wasserstoff bedeutet und $R^{14}$ 4-Pyridyl, 2-Phenyläthyl, 2-Piperidinoäthyl, Carboxymethyl, Aethoxycarbonylmethyl, Carbamoylmethyl, 1-Aethoxycarbonyläthyl, 1,4-Bis-(äthoxycarbonyl)-2-butyl oder 2-(5-Chlor-2-pyridincarboxamido)äthyl bedeutet.

6. Verbindung gemäss Anspruch 5, worin $R^{12}$ Aethyl, n-Propyl, Isopropyl, n-Nonyl oder Carbamoylmethyl bzw. $R^{13}$ Wasserstoff und $R^{14}$ Wasserstoff, Aethoxycarbonylmethyl oder 1,4-Bis-(äthoxycarbonyl)-2-butyl bedeuten.

7. Verbindungen gemäss einem der Ansprüche 1 bis 6, worin $R^7$ Methyl, 3-Acetylaminopropyl, Amino oder p-Methoxyphenyl bedeutet.

8. Verbindungen gemäss Anspruch 7, worin $R^7$ Methyl oder 3-Acetylaminopropyl bedeutet.

9. Verbindungen gemäss einem der Ansprüche 1 bis 8, worin $R^8$ und $R^9$ entweder beide Wasserstoff oder beide Methyl bedeuten.

10. Verbindungen gemäss Anspruch 9, worin $R^8$ und $R^9$ beide Wasserstoff bedeuten.

11. Verbindungen gemäss einem der Ansprüche 1 bis 10, worin $R^{10}$ und $R^{11}$ zusammen Oxo bedeuten.

12. N-[2-(4-Acetamidobutyryl)-5-chlorhydrocinnamoyl]glycinäthylester,.

13. 2-[4-(4-Acetamidobutyramido)butyryl]-5-chlorhydrozimtsäureäthylester.

14. 2-[4-(4-Acetamidobutyramido)butyryl]-5-chlorhydrozimtsäureamid.

15. 2-(4-Acetamidobutyryl)-5-chlorhydrozimtsäureisopropylester.

16. 2-(4-Acetamidobutyryl)-5-chlorhydrozimtsäureäthylester.

17. 2-[2-(4-Acetamidobutyryl)-5-chlorhydrocinnamoyl]-L-glutaminsäurediäthylester.

18. 2-(4-Acetamidobutyryl)-5-chlorhydrozimtsäurecarbamoylmethylester.

19. 2-(4-Acetamidobutyryl)-5-chlorhydrozimtsäureamid.

20. 2-(4-Acetamidobutyryl)-5-chlorhydrozimtsäurepropylester.

21. 2-(4-Acetamidobutyryl)-N-(carbamoylmethyl)-5-chlorhydrozimtsäuremid.

22. 2-(4-Acetamidobutyryl)-5-chlorhydrozimtsäurenonylester.

23. Verbindungen gemäss einem der Ansprüche 1 bis 22 zur Anwendung als therapeutische Wirkstoffe.

24. Verbindungen gemäss einem der Ansprüche 1 bis 22 zur Anwendung als der cerebralen Insuffizienz entgegenwirkende bzw. cognitive Funktionen verbessernde therapeutische Wirkstoffe.

25. Verfahren zur Herstellung von Verbindungen gemäss einem der Ansprüche 1 bis 22, dadurch gekennzeichnet, dass man

a) ein Benzazecindion der allgemeinen Formel

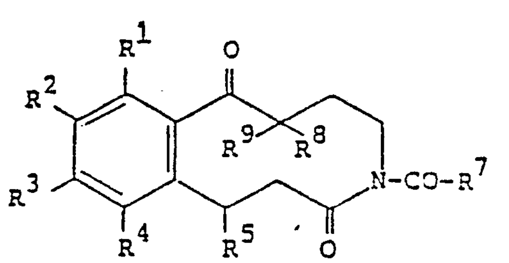

II

worin $R^1$, $R^2$, $R^3$, $R^4$, $R^5$, $R^7$, $R^8$ und $R^9$ die in Anspruch 1 angegebene Bedeutung besitzen, in Gegenwart einer Verbindung der allgemeinen Formel

$HOR^{12'}$ III

worin $R^{12'}$ Wasserstoff oder $(C_1-C_{10})$-Alkyl bedeutet, mit einer Säure behandelt; oder

b) eine Verbindung der in Anspruch 1 definierten Formel I, worin $R^6$ einen Rest der Formel (a) und $R^{12}$ Wasserstoff bedeuten, oder ein reaktionsfähiges Derivat davon, zu einer entsprechenden Verbindung der Formel I, worin $R^6$ einen Rest der Formel (a) bedeutet und $R^{12}$ von Wasserstoff verschieden ist, verestert; oder

c) eine Verbindung der Formel I, worin $R^6$ einen Rest der Formel (a) und $R^{12}$ Wasserstoff bedeuten, oder ein reaktionsfähiges Derivat davon, mit einer Verbindung der allgemeinen Formel

$HNR^{13}R^{14'}$ IV

worin $R^{13}$ die in Anspruch 1 angegebene Bedeutung besitzt und $R^{14'}$ die in Anspruch 1 für $R^{14}$ angegebene Bedeutung besitzt, aber nicht Carboxy-$(C_1-C_4)$-alkyl ist,
umsetzt oder eine Verbindung der Formel I, worin $R^6$ einen Rest der Formel (b) und $R^{14}$ Carboxy-$(C_1-C_4)$-alkyl bedeuten, oder ein reaktionsfähiges Derivat davon, mit Ammoniak umsetzt; oder

d) eine Verbindung der Formel I, worin $R^{10}$ und $R^{11}$ zusammen Oxo bedeuten, reduziert; oder

e) eine Verbindung der Formel I, worin $R^6$ einen Rest der Formel (b) und $R^{14}$ $(C_1-C_4)$-Alkoxycarbonyl-$(C_1-C_4)$-alkyl bedeuten, zu einer entsprechenden Verbindung der Formel I, worin $R^6$ einen Rest der Formel (b) und $R^{14}$ Carboxy-$(C_1-C_4)$-alkyl bedeuten, hydrolysiert; oder

f) eine basische Verbindung der Formel I mittels einer Säure bzw. eine saure Verbindung der Formel I mittels einer Base in ein pharmazeutisch anwendbares Salz überführt.

26. Arzneimittel, enthaltend eine Verbindung gemäss einem der Ansprüche 1 bis 22 und ein therapeutisch inertes Excipiens.

27. Der cerebralen Insuffizienz entgegenwirkendes bzw. cognitive Funktionen verbesserndes Arzneimittel, enthaltend eine Verbindung gemäss einem der Ansprüche 1 bis 22 und ein therapeutisch inertes Excipiens.

28. Verwendung von Verbindungen gemäss einem der Ansprüche 1 bis 22 bei der Bekämpfung bzw. Verhütung von Krankheiten bzw. bei der Verbesserung der Gesundheit.

29. Verwendung von Verbindungen gemäss einem der Ansprüche 1 bis 22 bei der Bekämpfung bzw. Verhütung der cerebralen Insuffizienz bzw. bei der Verbesserung cognitiver Funktionen.

30. Verwendung von Verbindungen gemäss einem der Ansprüche 1 bis 22 zur Herstellung von Arzneimitteln für die Bekämpfung bzw. Verhütung der cerebralen Insuffizienz bzw. für die Verbesserung cognitiver Funktionen.

Patentansprüche für die folgenden Vertragsstaaten: GR, ES.

1. Verfahren zur Herstellung von Hydrozimtsäurederivaten der allgemeinen Formel

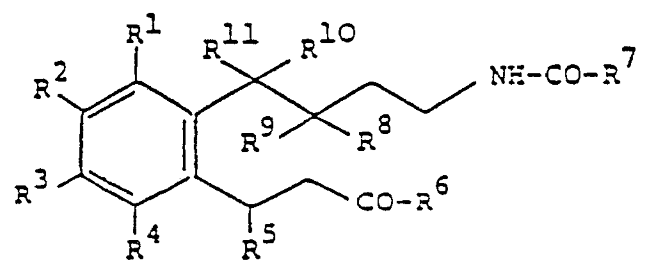

I

worin
- von den Symbolen $R^1$ bis $R^4$ eines oder zwei Halogen oder Methoxy und die übrigen Wasserstoff;
- $R^5$ Wasserstoff oder Phenyl;
- $R^6$ einen Rest der Formel $-OR^{12}$ (a) oder $-NR^{13}R^{14}$ (b);
- $R^7$ $(C_1-C_4)$-Alkyl, $(C_2-C_5)$-Alkanoylamino-$(C_2-C_5)$-alkyl, Amino oder $(C_1-C_4)$-Alkoxyphenyl;
- $R^8$ und $R^9$ je Wasserstoff oder $(C_1-C_4)$-Alkyl;

- $R^{10}$ Wasserstoff und $R^{11}$ Hydroxy oder $R^{10}$ und $R^{11}$ zusammen Oxo;
- $R^{12}$ Wasserstoff, $(C_1$-$C_{10})$-Alkyl, Pyridylmethyl oder Carbamoylmethyl;
- $R^{13}$ Wasserstoff, $(C_1$-$C_4)$-Alkyl und $R^{14}$ Wasserstoff, $(C_1$-$C_4)$-Alkyl, Pyridyl, Phenyl-$(C_1$-$C_4)$-alkyl, Carboxy-$(C_1$-$C_4)$-alkyl, Carbamoyl-$(C_1$-$C_4)$-alkyl, $(C_1$-$C_4)$-Alkoxycarbonyl-$(C_1$-$C_4)$-alkyl, Di-$(C_1$-$C_4)$-alkoxycarbonyl-$(C_2$-$C_5)$-alkyl, Piperidino-$(C_2$-$C_4)$-alkyl oder Halopyridincarboxamido-$(C_2$-$C_4)$-alkyl oder $R^{13}$ und $R^{14}$ zusammen mit dem Stickstoffatom 4-$(C_1$-$C_4)$-Alkyl-piperazin-1-yl bedeuten,

sowie pharmazeutisch anwendbaren Salzen von basischen Verbindungen der Formel I mit Säuren bzw. von sauren Verbindungen der Formel I mit Basen, dadurch gekennzeichnet, dass man

a) ein Benzazecindion der allgemeinen Formel

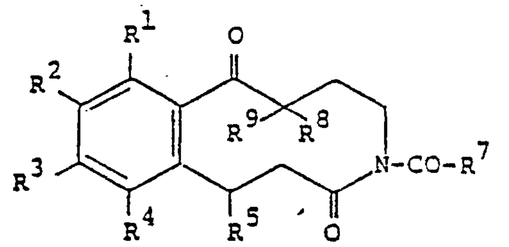

II

worin $R^1$, $R^2$, $R^3$, $R^4$, $R^5$, $R^7$, $R^8$ und $R^9$ obige Bedeutung besitzen, in Gegenwart einer Verbindung der allgemeinen Formel

$HOR^{12'}$ III

worin $R^{12'}$ Wasserstoff oder $(C_1$-$C_{10})$-Alkyl bedeutet, mit einer Säure behandelt; oder

b) eine Verbindung der obigen Formel I, worin $R^6$ einen Rest der Formel (a) und $R^{12}$ Wasserstoff bedeuten, oder ein reaktionsfähiges Derivat davon, zu einer entsprechenden Verbindung der Formel I, worin $R^6$ einen Rest der Formel (a) bedeutet und $R^{12}$ von Wasserstoff verschieden ist, verestert; oder

c) eine Verbindung der Formel I, worin $R^6$ einen Rest der Formel (a) und $R^{12}$ Wasserstoff bedeuten, oder ein reaktionsfähiges Derivat davon, mit einer Verbindung der allgemeinen Formel

$HNR^{13}R^{14'}$ IV

worin $R^{13}$ obige Bedeutung besitzt und $R^{14'}$ die oben für $R^{14}$ angegebene Bedeutung besitzt, aber nicht Carboxy-$(C_1$-$C_4)$-alkyl ist,
umsetzt oder eine Verbindung der Formel I, worin $R^6$ einen Rest der Formel (b) und $R^{14}$ Carboxy-$(C_1$-$C_4)$-alkyl bedeuten, oder ein reaktionsfähiges Derivat davon, mit Ammoniak umsetzt; oder

d) eine Verbindung der Formel I, worin $R^{10}$ und $R^{11}$ zusammen Oxo bedeuten, reduziert; oder

e) eine Verbindung der Formel I, worin $R^6$ einen Rst der Formel (b) und $R^{14}$ $(C_1$-$C_4)$-Alkoxycarbonyl-$(C_1$-$C_4)$-alkyl bedeuten, zu einer entsprechenden Verbindung der Formel I, worin $R^6$ einen Rest der Formel (b) und $R^{14}$ Carboxy-$(C_1$-$C_4)$-alkyl bedeuten, hydrolysiert; oder

f) eine basische Verbindung der Formel I mittels einer Säure bzw. eine saure Verbindung der Formel I mittels einer Base in ein pharmazeutisch anwendbares Salz überführt.

2. Verfahren gemäss Anspruch 1, dadurch gekennzeichnet, dass man eine Verbindung der Formel I herstellt, worin $R^4$ Wasserstoff bedeutet und entweder $R^1$ Chlor und $R^2$ und $R^3$ Wasserstoff oder $R^1$ Wasserstoff und $R^2$ und $R^3$ Chlor oder $R^1$ und $R^2$ Wasserstoff und $R^3$ Fluor, Chlor oder Methoxy bedeuten.

3. Verfahren gemäss Anspruch 2, dadurch gekennzeichnet, dass man eine Verbindung der Formel I herstellt, worin $R^3$ Chlor und $R^1$, $R^2$ und $R^4$ Wasserstoff bedeuten.

4. Verfahren gemäss einem der Ansprüche 1 bis 3, dadurch gekennzeichnet, dass man eine Verbindung der Formel I herstellt, worin $R^5$ Wasserstoff bedeutet.

5. Verfahren gemäss einem der Ansprüche 1 bis 4, dadurch gekennzeichnet, dass man eine Verbindung der Formel I herstellt, worin $R^5$ einen Rest der in Anspruch 1 definierten Formel (a) oder (b) bedeutet, wobei $R^{12}$ Wasserstoff, Methyl, Aethyl, n-Propyl, Isopropyl, n-Butyl, tert.-Butyl, n-Nonyl, 5-Nonyl, 3-Pyridylmethyl oder Carbamoylmethyl bedeutet bzw. entweder $R^{13}$ und $R^{14}$ beide Wasserstoff oder beide Aethyl oder zusammen mit dem Stickstoffatom 4-Methylpiperazin-1-yl bedeuten oder $R^{13}$ Wasserstoff bedeutet und $R^{14}$ 4-Pyridyl, 2-Phenyläthyl, 2-Piperidinoäthyl, Carboxymethyl, Aethoxycarbonylmethyl, Carbamoylmethyl, 1-Aethoxycarbonyläthyl, 1,4-Bis-(äthoxycarbonyl)-2-butyl oder 2-(5-Chlor-2-pyridincarboxamido)äthyl bedeutet.

6. Verfahren gemäss Anspruch 5, worin dadurch gekennzeichnet, dass man eine Verbindung der Formel I herstellt, $R^{12}$ Aethyl, n-Propyl, Isopropyl, n-Nonyl oder Carbamoylmethyl bzw. $R^{13}$ Wasserstoff und $R^{14}$ Wasserstoff, Aethoxycarbonylmethyl oder 1,4-Bis-(äthoxycarbonyl)-2-butyl bedeuten.

7. Verfahren gemäss einem der Ansprüche 1 bis 6, dadurch gekennzeichnet, dass man eine Verbindung der Formel I herstellt, worin $R^7$ Methyl, 3-Acetylaminopropyl, Amino oder p-Methoxyphenyl bedeutet.

8. Verfahren gemäss Anspruch 7, dadurch gekennzeichnet, dass man eine Verbindung der Formel I herstellt, worin $R^7$ Methyl oder 3-Acetylaminopropyl bedeutet.

9. Verfahren gemäss einem der Ansprüche 1 bis 8, dadurch gekennzeichnet, dass man eine Verbindung der Formel I herstellt, worin $R^8$ und $R^9$ entweder beide Wasserstoff oder beide Methyl bedeuten.

10. Verfahren gemäss Anspruch 9, dadurch gekennzeichnet, dass man eine Verbindung der Formel I herstellt, worin $R^8$ und $R^9$ beide Wasserstoff bedeuten.

11. Verfahren gemäss einem der Ansprüche 1 bis 10, dadurch gekennzeichnet, dass man eine Verbindung der Formel I herstellt, worin $R^{10}$ und $R^{11}$ zusammen Oxo bedeuten.

12. Verfahren gemäss Anspruch 1, dadurch gekennzeichnet, dass man N-[2-(4-Acetamidobutyryl)-5-chlorhydrocinnamoyl]glycinäthylester herstellt.

13. Verfahren gemäss Anspruch 1, dadurch gekennzeichnet, dass man 2-[4-(4-Acetamidobutyramido)-butyryl]-5-chlorhydrozimtsäureäthylester herstellt.

14. Verfahren gemäss Anspruch 1, dadurch gekennzeichnet, dass man 2-[4-(4-Acetamidobutyramido)-butyryl]-5-chlorhydrozimtsäureamid herstellt.

15. Verfahren gemäss Anspruch 1, dadurch gekennzeichnet, dass man 2-(4-Acetamidobutyryl)-5-chlorhydrozimtsäureisopropylester herstellt.

16. Verfahren gemäss Anspruch 1, dadurch gekennzeichnet, dass man 2-(4-Acetamidobutyryl)-5-chlorhydrozimtsäureäthylester herstellt.

17. Verfahren gemäss Anspruch 1, dadurch gekennzeichnet, dass man 2-[2-(4-Acetamidobutyryl)-5-chlorhydrocinnamoyl]-L-glutaminsäurediäthylester herstellt.

18. Verfahren gemäss Anspruch 1, dadurch gekennzeichnet, dass man 2-(4-Acetamidobutyryl)-5-chlorhydrozimtsäurecarbamoylmethylester herstellt.

19. Verfahren gemäss Anspruch 1, dadurch gekennzeichnet, dass man 2-(4-Acetamidobutyryl)-5-chlorhydrozimtsäureamid herstellt.

20. Verfahren gemäss Anspruch 1, dadurch gekennzeichnet, dass man 2-(4-Acetamidobutyryl)-5-chlorhydrozimtsäurepropylester herstellt.

21. Verfahren gemäss Anspruch 1, dadurch gekennzeichnet, dass man 2-(4-Acetamidobutyryl)-N-(carbamoylmethyl)-5-chlor-hydrozimtsäureamid herstellt.

22. Verfahren gemäss Anspruch 1, dadurch gekennzeichnet, dass man 2-(4-Acetamidobutyryl)-5-chlorhydrozimtsäurenonylester herstellt.

23. Verfahren zur Herstellung von Arzneimitteln, insbesondere zur Verwendung bei der Bekämpfung bzw. Verhütung der cerebralen Insuffizienz bzw. bei der Verbesserung cognitiver Funktionen, dadurch gekennzeichnet, dass man eine oder mehrere der Verbindungen der in Anspruch 1 definierten Formel I und/oder der pharmazeutisch annehmbaren Salze dieser Verbindungen und erwünschtenfalls einen oder mehrere andere therapeutisch wirksame Stoffe zusammen mit einem oder mehreren therapeutisch inerten Excipientien in eine galenische Darreichungsform bringt.

24. Verwendung von Verbindungen der in Anspruch 1 definierten Formel I und von pharmazeutisch annehmbaren Salzen dieser Verbindungen zur Herstellung von Arzneimitteln für die Bekämpfung bzw. Verhütung der cerebralen Insuffizienz bzw. für die Verbesserung cognitiver Funktionen.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 88111347.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A - 2 141 598 (HOFFMANN-LA ROCHE)<br>* Anspruch 1 *<br>— | | C07C103/46<br>C07C103/30<br>C07C103/84<br>C07C103/49<br>C07D225/06<br>C07D213/30<br>C07D213/75<br>C07D295/12<br>C07C127/17<br>A61K31/165<br>A61K31/44<br>A61K31/445 |
| A,P | EP - A - 2 30967 (HOFFMANN-LA ROCHE)<br>* Ansprüche 1,26,27 *<br>— | 25,30 | |

RECHERCHIERTE SACHGEBIETE (Int. Cl.4)

C07C103/00
C07D225/00
C07D213/00
C07D295/00
C07C127/00
A61K31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-27,30

Unvollständig recherchierte Patentansprüche: –

Nicht recherchierte Patentansprüche: 28,29

Grund für die Beschränkung der Recherche:

Artikel 52(4) EPÜ

| Recherchenort | Abschlußdatum der Recherche | Prüfer |
|---|---|---|
| BERLIN | 27.09.1988 | J. RUFET |

KATEGORIE DER GENANNTEN DOKUMENTEN

X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& Mitglied der gleichen Patentfamilie, übereinstimmendes Dokument